# EUROPEAN PATENT SPECIFICATION

(11) **EP 2 170 391 B1**
(45) Date of publication and mention of the grant of the patent: **18.01.2017**
(21) Application number: 08771342.6
(22) Date of filing: 18.06.2008
(51) Int. Cl.: A61K 39/095, A61K 39/09

(54) **MODIFIED POLYSACCHARIDES FOR CONJUGATE VACCINES**
MODIFIZIERTE POLYSACCHARIDE FÜR KONJUGAT-IMPFSTOFFE
POLYSACCHARIDES MODIFIÉS POUR VACCINS CONJUGUÉS

(30) Priority: 20.06.2007 US 945226 P
(43) Date of publication of application: 07.04.2010
(73) Proprietor: Pfizer Ireland Pharmaceuticals, Ringaskiddy Cork (IE)
(72) Inventor: MICHON, Francis, Bethesda, Maryland 20814 (US); SARKAR, Arun, North Potomac, Maryland 20878 (US)
(74) Representative: Williams, Gareth Owen
(86) International application number: PCT/US2008/067315
(87) International publication number: WO 2008/157590

(56) References cited:
- WO-A1-96/40239
- WO-A2-2006/002402
- US-A- 5 576 002
- US-A- 5 866 135
- US-A1- 2006 035 284
- US-B1- 6 350 449
- US-B1- 6 350 861

## Description

### 1. FIELD OF THE INVENTION

The present invention relates to methods of manufacture of immunogenic glycoconjugates, in particular for use in pharmaceutical compositions for inducing a therapeutic immune response in a subject. The immunogenic glycoconjugates of the invention comprise one or more oligosaccharides or polysaccharides that are conjugated to one or more carrier proteins via an active aldehyde group. Accordingly, the invention provides methods of making (i) unsaturated microbial N-acyl derivative oligosaccharides or polysaccharides; (ii) novel conjugates of unsaturated N-acyl derivatives; and (iii) glycoconjugate compositions comprising conjugate molecules of fragments of microbial unsaturated N-acyl derivatives that serve as a covalent linker to one or more proteins. The invention further encompasses the immunogenic glycoconjugates pharmaceutical compositions for the prevention or treatment of an infectious disease.

### 2. BACKGROUND OF THE INVENTION

Microbial infections caused by gram-positive bacteria such as Streptococcus, Staphylococcus, Enterococcus, Bacillus, Corynebacterium, Listeria, Erysipelothrix, and Clostridium and by gram-negative bacteria such as Haemophilus, Shigella, Vibrio cholerae, Neisseria and certain types of Escherichia coli cause serious morbidity throughout the world. Streptococci, for example, are a large and varied genus of gram-positive bacteria which have been ordered into several groups based on the antigenicity and structure of their cell wall polysaccharide (Lancefield, R. C. 1933. A serological differentiation of human and other groups of hemolytic streptococci. J.Exp.Med. 57:571-595. Lancefield, R. C. 1938. A micro-precipitin technique for classifying hemolytic streptococci and improved methods for producing antigen. Proc.Soc.Exp.Biol.and Med. 38:473-478).

Group B Streptococci, for example, are classified into several different types based on capsular polysaccharide, such as types Ia, Ib, II, III, IV, V, VI, VII, and VIII, which account for most of the pathogenicity. Similar to findings with many other human bacterial pathogens, capsular polysaccharides of group B streptococci, when used in vaccines, can provide effective protection against infections with these bacteria (See Wessels, et al. 1990. Immunogenicity in animals of a polysaccharide-protein conjugate vaccine against type III group B Streptococcus. J. Clin. Invest. 86:1428-1433. Wessels, et al. 1993. Stimulation of protective antibodies against type Ia and lb group B streptococci by a type Ia polysaccharide-tetanus toxoid conjugate vaccine. Infect. Immun. 61:4760-4766).

Gram-negative bacteria also are a significant cause of morbidity and mortality. Until the recent development and use of polysaccharide-protein vaccines directed against *Haemophilus influenzae* type b bacteria (Hib), Hib bacterial infections were responsible for many cases of mental retardation in infants.

Infants and young children typically have poor immunogenic response to polysaccharide antigens. These responses are characterized as being T cell independent and therefore are not associated with important attributes such as memory, isotype switching, or affinity maturation, which are necessary for conferring long term immunologic protection against subsequent infection. To circumvent this lack of an effective immunogenic response in infants and young children to polysaccharides, the field has worked towards developing approaches for converting the T cell independent response to a T cell dependent response by covalently coupling polysaccharide bacterial antigens to a carrier protein to form a conjugate molecule. See, Jennings et al. U.S. Pat. No. 4,356,170).

Adjuvants are substances that augment the immune response to antigens and, therefore, have been used in many vaccines and vaccine candidates. The immune stimulatory effect of adjuvants is not antigen specific, as they boost immune responses towards many different types of antigens. The only adjuvants currently approved for human use by the FDA are aluminum salts, but many adjuvants used in animal vaccinations and in newer vaccine candidates are microbial in origin (White, R. G., 1976. The adjuvant effect of microbial products on the immune response. Ann.Rev.Microbiol. 30:579-595; hereby incorporated by reference in its entirety). Such adjuvants include, Freund's adjuvant, Corynebacterium parvum, muramyl dipetide, tetanus toxoid, etc.

Conjugation of a polysaccharide to a carrier protein can effectively make that polysaccharide more immunogenic. The carrier proteins known in the art include, tetanus toxin/toxoid, CRM₁₉₇, outer membrane proteins from gram negative bacteria, for example, high molecular weight proteins, P6 and P4 from nontypeable *Haemophilus influenzae,* CD and USPA from *Moraxella catarrhalis*, diphtheria toxin/toxoid, detoxified *Pseudomonas aeruginosa toxin* A, cholera toxin/toxoid, pertussis toxin/toxoid, *Clostridium perfringens* exotoxins/toxoid, hepatitis B surface antigen, hepatitis B core antigen, rotavirus VP 7 protein, or respiratory syncytial virus F and G protein.

Tetanus toxoid has been used for decades in this capacity as a carrier, and its safety profile has been established.

Capsular polysaccharides (CP) conjugate vaccines targeting a variety of bacterial infections are currently under development and clinical evaluation. The inclusion of multiple CP serotypes combined in a single injection is currently under study. The combination of CP conjugate vaccines into a single multivalent injection, however, can result in competition among the different components and adversely affect the immunogenicity of any individual conjugate (Fattom et al., 1999, Vaccine 17: 126-33).

Various procedures have been described in the art for conjugating capsular polysaccharides to proteins. Conjugation of a polysaccharide to a carrier protein can effectively make that polysaccharide more immunogenic (Robbins, J. B. and R. Schneerson. 1990. Polysaccharide-protein conjugates: A new generation of vaccines. J. Infect. Dis. 161:821-832). For another review, see Contributions to Microbiology and Immunology, vol 10, Conjugate Vaccines, volume editions J. M. Cruse and R. E. Lewis, Jr., 1989. In one method, polysaccharides are subjected to mild acid hydrolysis to produce reducing end groups capable of reacting with protein to form a covalent bond (Anderson, P. A., 1983, Infect. Immun., 39:233-238).
However, the terminal sugar groups which participate in conjugating to protein exist in equilibrium between a hemiacetal and aldehyde and therefore couple to protein with poor efficiency. To overcome the poor reactivity of the terminal reducing sugar, the art turned to mild oxidation to introduce stable aldehyde groups at terminal positions of polysaccharides used to conjugate to protein (see Jennings et al. U.S. Pat. No. 4,356,170, supra).

Other available methods, for example, activation through IO₄⁻ oxidation of some polysaccharides, may generate only one active site per polysaccharide molecule and consequent coupling to carrier protein generates single-ended glycoconjugate vaccines. This arrangement is found in, for example, glyconjugate vaccines for *Neisseria meningitidis* type C, type B, and Streptococcus group A. However, a single active site per molecule limits the degree of immunogenicity enhancement.

WO9640239 discloses modified N. meningitidis polysaccharides wherein a protein is conjugated to the terminus of the polysaccharide.

### 3. SUMMARY OF THE INVENTION

The invention to which the present specification relates is as set out in the claims appended to this description.

The present invention provides polysaccharides with multiple active sites that permit the generation of immunogenic cross-linked glycoconjugate vaccines through conjugation to a suitable carrier protein. Moreover, the method also is applicable to any polysaccharide containing an amino sugar, a clear advantage over existing IO₄⁻ oxidation method, which requires two adjacent, *i.e.,* vicinal, free hydroxy (-OH) group in the sugar chain.

The present invention relates to methods of manufacture of immunogenic glycoconjugates, in particular for use in pharmaceutical compositions for inducing a therapeutic immune response in a subject. The immunogenic glycoconjugates of the invention comprise one or more oligosaccharides or polysaccharides that are conjugated to one or more carrier proteins via an active aldehyde group. Unlike previous conjugation methods known in the art, the present invention provides methods that are applicable to any polysaccharide that contains at least one amino sugar, which methods also produce well defined, soluble conjugates that maintain antigenicity.

The disclosure provides methods of making an immunogenic glycoconjugate comprising the steps of: deacetylating at least one N-acetyl group in an oligosaccharide or polysaccharide comprising one or more amino sugars, forming an oligosaccharide or polysaccharide having at least one, preferably multiple, amino sugar(s) comprising a primary amino group; substituting at least one of the primary amino groups with an N-acyl moiety that comprises an unsaturated alkyl moiety at least 4 carbons in length, thereby generating an oligosaccharide or polysaccharide comprising at least one, preferably multiple, active site(s) at the site of unsaturation of the one or more alkyl moieties; contacting the compound with an oxidizing agent, generating an active aldehyde (-CHO) group at the one or more unsaturated sites on the oligosaccharide or polysaccharide; and conjugating the compound with a carrier protein, thereby generating an immunogenic glycoconjugate.

In alternate embodiments, the disclosure provides methods of making an immunogenic glycoconjugate comprising the steps of: substituting at least one N-acetyl group in an oligosaccharide or polysaccharide comprising one or more amino sugars with an N-acyl moiety that comprises an unsaturated alkyl moiety at least 4 carbons in length, thereby generating an oligosaccharide or polysaccharide comprising at least one, preferably multiple, active site(s) at the site of unsaturation of the one or more alkyl moieties; contacting the compound with an oxidizing agent, generating an active aldehyde (-CHO) group at the one or more unsaturated sites of the alkyl moieties on the oligosaccharide or polysaccharide; and conjugating the compound with a carrier protein, thereby generating an immunogenic glycoconjugate.

In accordance with the methods of the disclosure the N-acyl moiety comprises an unsaturated alkyl moiety. In certain embodiments, the unsaturated alkyl moiety is a C₃ C₄, C₅, C₆, C₇, C₇, C₈, C₉, C₁₀, or a C₁₁ moiety, and may comprise one or more double bonds within the carbon backbone chain. In certain embodiments, the unsaturated alkyl moiety comprises only one double bond, *i.e*., one site of unsaturation. In other embodiments, the N-acyl moiety is an N-pentenoyl moiety. The unsaturated N-acyl moiety may comprise any unsaturated alkyl moiety described herein or known in the art to be suitable for oxidation to an active aldehyde at the site of unsaturation and subsequent conjugation to a carrier protein, *e.g*., an unsaturated acyl anhydride (*e.g*., pentenoic anhydride) or an unsaturated acyl halide (*e.g*., pentenoyl chloride, acroloyl chloride).

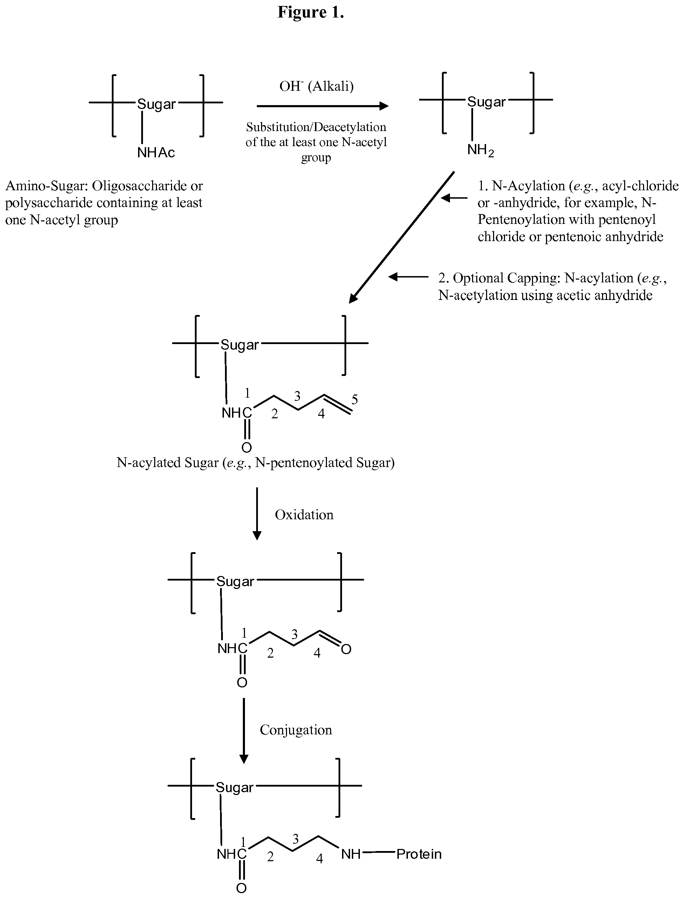

Oxidation of the molecules of the disclosure is preferably limited to oxidation of the double bonds of the unsaturated N-Acyl moiety (*i.e*., the site(s) of unsaturation of the unsaturated alkyl moiety) and is, therefore, performed under mild conditions as defined herein or as is known in the art, *e.g*., oxidation using periodate at a pH range of 4-9.5 as is known in the art. Because of the mild oxidation conditions, sites of unsaturation located too near the acyl group will fail to be oxidized and/or fail to form an active aldehyde group to allow subsequent conjugation to a carrier protein. Accordingly, for use in the methods of the invention, the site of unsaturation (*i.e*., the location of the double bond) of the unsaturated alkyl group is neither between carbons 1 and 2 nor between carbons 2 and 3 of the unsaturated alkyl group (see, *e.g*., FIG. 1 for the numbering of the alkyl group as accepted in the art and as used herein). In certain embodiments, the unsaturated alkyl moiety comprises only one double bond. In other embodiments, the unsaturated alkyl moiety comprises a double bond between the terminal two carbons of the backbone carbon chain.

The disclosure further provides an immunogenic glycoconjugate comprising: a) at least one oligosaccharide or polysaccharide comprising one or more amino sugars prepared by
i) deacetylating the N-acetyl group of said one or more amino sugars; ii) substituting the resulting primary amino group of the amino sugar with an N-acyl group comprising an unsaturated alkyl group of at least 4 carbons; and iii) oxidizing said unsaturated alkyl group to generate an alkyl group of at least 3 carbons with an active aldehyde group at the terminus of the backbone chain; and b) a carrier protein conjugated thereto. In accordance with the methods of the invention, the site of unsaturation, *i.e*., the double bond, of the unsaturated alkyl group is neither between carbons 1 and 2 nor between carbons 2 and 3 of the backbone chain (see, *e.g*., FIG. 1 for carbon numbering.) In preferred embodiments, the unsaturated alkyl chain is an at least 5 carbons in length. In other embodiments, the site of unsaturation or the alkyl chain prior to oxidation is between the terminal two carbons of the unsaturated alkyl chain. According to the methods of the invention, the carrier protein is conjugated to the at least one polysaccharide or oligosaccharide via the active aldehyde group by any method known in the art and/or described herein.

The disclosure also provides an immunogenic glycoconjugate comprising: a) at least one oligosaccharide or polysaccharide comprising one or more amino sugars comprising one or more N-acetyl groups, wherein said one or more N-acetyl groups have been substituted with an N-acyl group comprising an alkyl group of at least 3 carbons comprising an active aldehyde group at the terminus of said alkyl group backbone and b) a carrier protein conjugated thereto. In preferred embodiments, the alkyl chain comprising the active aldehyde group is at least 4 carbons in length. According to the methods of the invention, the carrier protein is conjugated to the at least one polysaccharide or oligosaccharide via the active aldehyde group by any method known in the art, e.g., reductive amination, and/or described herein.

According to disclosure the at least one N-acetyl group on the one or more amino sugars of the polysaccharide or oligosaccharide is substituted to form the compound as follows (Formula I): 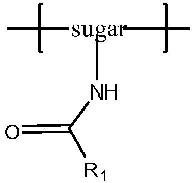 wherein the R₁ is an unsaturated C₃ C₄, C₅, C₆, C₇, C₈, C₉, C₁₀, or C₁₁ alkyl moiety and the ⁅sugar⁆ represents the said one or more amino sugar of the polysaccharide or oligosaccharide. In certain embodiments, said one or more amino sugar is a component of the repeating unit of the polysaccharide or oligosaccharide. In other embodiments, the said one or more amino sugar is not a component of the repeating unit of the polysaccharide or oligosaccharide. In specific embodiments, R₁ is C₃ or C₄, *e.g*., such that an N-butenoyl group or N-pentenoyl group is formed, respectively. In other embodiments, R₁ contains only one double bond, *i.e*., site of unsaturation, which double bond is located between the terminal two carbons of the alkyl backbone chain.

The unsaturated N-acyl moiety serves as a linking moiety for conjugating the oligosaccharide or polysaccharide with the carrier protein via oxidation of the site of unsaturation to an active aldehyde group and subsequent conjugation to said protein. Oxidation of the site of unsaturation of the alkyl moiety may be performed by any method described herein and/or known in the art, *e.g.* with periodate. Because oxidation can cleave the polysaccharide chain, the oxidation step encompassed by the present invention is performed only under mild conditions, *e.g*., low temperature (*e.g*., about 4 °C to about 27 °C) at a pH range of about 4 to about 9.5. Due to mild oxidative conditions, for the site of unsaturation to be oxidized efficiently, the double bond of the unsaturated alkyl moiety cannot be between carbons 1 and 2, or between carbons 2 and 3 of the unsaturated alkyl backbone chain (see, *e.g*., FIG. 1 for carbon numbering). In a non-limiting example in accordance with this embodiment, wherein R₁ is an unsaturated C₃ moiety (forming an N-butenoyl group in Formula 1), the N-acyl group comprises a double bond between carbons 3 and 4 of the alkyl backbone chain. In other embodiments, wherein R₁ comprises a chain of greater than 3 carbons, the site of unsaturation may be at positions greater than carbon 4.

In accordance with the products and methods of the disclosure subsequent to oxidation and conjugation, the protein and the oligosaccharide or polysaccharide of the glycoconjugate are covalently linked through a linkage as shown below (boxed; Formula II): 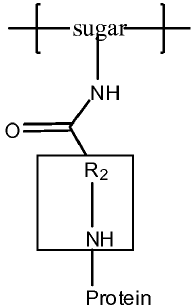 wherein R₂ is a saturated C₂ C₃, C₄, C₅, C₆, C₇, C₈, C₉ or C₁₀ alkyl moiety, and wherein NH (boxed) belongs to one of the primary NH₂ groups of the protein, e.g., lysinyl or arginyl residues. The ⁅sugar⁆ of Formula II represents the said one or more amino sugar of the polysaccharide or oligosaccharide. In certain embodiments, said one or more amino sugar is a component of the repeating unit of the polysaccharide or oligosaccharide. In other embodiments, the said one or more amino sugar is not a component of the repeating unit of the polysaccharide or oligosaccharide. R₂ of Formula II is derived from R₁ of Formula I. R₁ of Formula I (*i.e*., an unsaturated alkyl moiety) is selected such that subsequent to oxidation and conjugation, as is known in the art and/or described herein, the desired linking moiety R₂ or formula II (*i.e*., a saturated alkyl moiety) results.

In accordance with the methods of the invention, the N-acetyl of the one or more amino sugars of the polysaccharide or oligosaccharide may be linked to the amino sugar at any position, including 1, 2, 3, 4, or 5 of the sugar. The structures of amino sugars are well known in the art, and thus, the position of said linkage may be routinely determined. For example, where the amino sugar is GlcNAc, the N-acetyl group of the sugar is at carbon 2; where the amino sugar is sialic acid, the N-acetyl group of the sugar is at carbon 4.

In a specific embodiment, the N-acetyl group of the sugar molecule is substituted with an N-pentenoyl group, wherein the N-pentenoyl group serves as a linking moiety for conjugating the oligosaccharide or polysaccharide with the carrier protein, e.g. conjugation by reductive amination. In a specific embodiment, subsequent to oxidation and conjugation the link group is a saturated N-acyl group with a saturated C₄ backbone. In accordance with the methods of the invention, the N-acetyl group to be substituted may be that of any amino sugar, including, but not limited to, GlcNAc, ManNAc, GalNAc, and Sialic acid.

In certain embodiments of the invention, substitution of N-acetyl groups is carried out with an alkali.

The invention encompasses the use of any carrier protein known in the art and/or described herein that is suitable for use in immunogenic conjugate vaccines and that functions to convert a T cell independent immune response to a T cell dependent immune response. In certain embodiments, the carrier protein is a bacterial protein or fragment thereof, *e.g*., a bacterial toxin or toxoid or fragment thereof. Non-limiting examples of carrier proteins that may me used in accordance with the methods of the invention include tetanus toxin or toxoid, CRM₁₉₇, outer membrane proteins from gram negative bacteria, P6 and P4 from nontypeable *Haemophilus influenzae*, protein derived from *Haemophilus influenzae* Type B, CD and USPA from *Moraxella catarrhalis*, diphtheria toxin/toxoid, detoxified *Pseudomonas aeruginosa* toxin A, cholera toxin/toxoid, pertussis toxin/toxoid, *Clostridium perfringens* exotoxins/toxoid, hepatitis B surface antigen, hepatitis B core antigen, rotavirus VP7 protein, and respiratory syncytial virus F protein and G protein.

In certain embodiments, the oligosaccharide or polysaccharide used in accordance with the methods of the invention is a polysaccharide from a bacterium or antigenic fragment thereof. Such bacteria include, but are not limited to, *Streptococcus, Staphylococcus*, *Enterococcus*, *Bacillus*, *Corynebacterium*, *Listeria*, *Erysipelothrix*, *Clostridium*, *Shigella*, *Klebsiella*, *Vibrio cholerae*, *Neisseria*, and *Escherichia.* In related embodiments, the oligosaccharide or polysaccharide is a capsular polysaccharide derived from any capsular bacteria, for example, Group B Streptococci Type Ia, Ib, II, III, V, VI, or VIII; Group A *Streptococcus*; *Neisseria meningitidis* types B, C, Y, or W135; *S. pneumoniae* Types III, IV, or XIV; or *Escherichia coli* K1. In preferred embodiments, the oligosaccharide or polysaccharide is chosen such that is capable of inducing an immune response against the bacteria from which it is derived. In certain embodiments, the oligosaccharide or polysaccharide is not a naturally occurring oligosaccharide or polysaccharide, e.g., may be synthesized by any technique known in the art, or may be derived from naturally occurring compounds but modified such that the resultant oligosaccharide or polysaccharide is not found in nature. For example, the polysaccharide or oligosaccharide may be modified so as to increase the antigenicity, *e.g*., induce an increased immune response, against the wild-type polysaccharide or oligosaccharide relative. In accordance with the methods of the invention, useful polysaccharides comprise at least one antigenic epitope capable of inducing an immunospecific response. Such polysaccharides or oligosaccharides preferably contain at least 7 saccharide moieties, but may demonstrate activity with as few as two saccharide moieties. The polysaccharides or oligosaccharides may be unbranched or branched, and can have a molecular weight of from about 1000 to several million Daltons. In certain embodiments, the polysaccharides or oligosaccharides possess one or more chemical modifications. Non-limiting examples of chemical modification encompassed by the present invention include carboxylation, sulfonation, sulfated, and phosphated derivatives of polysaccharides, their salts, and mixtures thereof.

The present disclosure also encompasses the use of compositions, in particular pharmaceutical compositions, comprising one or more immuno-glycoconjugates at therapeutically effective concentrations for inducing an immune response in a subject. Induction of an immune response to the oligosaccharide or polysaccharide of the immuno-glycoconjugate is useful for the treatment and/or prevention of an infection by the pathogenic organism(s) from which the oligosaccharide or polysaccharide is derived. Accordingly, in certain embodiments, the glycoconjugate of the invention can be used as a vaccine for prophylaxis or as a therapeutic. In certain embodiments, an immunoglycoconjugate comprises a plurality of polysaccharides and/or oligosaccharides derived from more than one type or strain of bacteria, thereby inducing an immune response against more that one species/type or strain of bacteria. In other embodiments, the oligosaccharide or polysaccharide used in the manufacture of the immuno-glycoconjugate of the invention is synthesized to comprise multiple antigenic domains from multiple species/strains of bacteria and/or other pathogenic organisms, e.g., protozoa, such that a multi-valent immune response is generated on administration to a subject.

The invention also relates to immunogenic preparations for humans or animals, in particular to immunogenic compositions comprising one or more immuno-glycoconjugates of the invention. In certain embodiments, the immunogenic preparations comprise a plurality of immuno-glycoconjugates and/or a plurality of carrier proteins. Because the immuno-glycoconjugates can be engineered to comprise polysaccharides or oligosaccharides from multiple types or strains of bacteria and/or engineered to comprise a chimeric polysaccharide or oligosaccharide (*i.e*., a polysaccharide or oligosaccharide comprising epitopes from multiple types and/or strains of bacteria), immunogenic formulations of the invention can be engineered for prophylaxis or therapy against multiple bacterial types, strain variant and or strain variants. Many methods well known and routine in the art may be used to immunize a subject with the immunogenic formulations of the invention including, but not limited to, intranasal, intratrachial, oral, intradermal, intramuscular, intraperitoneal, intravenous and subcutaneous routes.

### 3.1 TERMINOLOGY

As used herein, the term "about" or "approximately" when used in conjunction with a number refers to any number within 1, 5 or 10% of the referenced number or within the experimental error typical of standard methods used for the measurement and/or determination of said number.

The term "carrier", "carrier protein", or "carrier polypeptide" are used interchangeably to refer to a polypeptide moiety to which the polysaccharide antigens are covalently linked. A carrier protein is often immunogenic and therefore may also contribute to the valency of the vaccine. Linkage to the carrier protein typically increases the antigenicity of the conjugated carbohydrate molecules. The carrier protein may be from the same target organism as the polysaccharides linked to it or may be from a different organism. For example, the carrier protein may be a bacterial protein, including, but not limited to, a bacterial toxin or toxoid. In preferred embodiments, the carrier protein is selected such that it functions to convert a T-cell independent immune response to a T cell dependent immune response.

As used herein, the term "in combination" in the context of the administration of (a) therapy(ies) to a subject, refers to the use of more than one therapy (*e.g*., more than one prophylactic agent and/or therapeutic agent). The use of the term "in combination" does not restrict the order in which therapies (*e.g*., prophylactic and/or therapeutic agents) are administered to a subject. A first therapy (*e.g*., a first prophylactic or therapeutic agent) can be administered prior to (*e.g*., 5 minutes, 15 minutes, 30 minutes, 45 minutes, 1 hour, 2 hours, 4 hours, 6 hours, 12 hours before), concomitantly with, or subsequent to *(e.g.,* 5 minutes, 15 minutes, 30 minutes, 45 minutes, 1 hour, 2 hours, 4 hours, 6 hours, 12 hours) the administration of a second therapy (*e.g*., a second prophylactic or therapeutic agent) to a subject. In specific embodiments, the immunogenic glycoconjugates of the invention may be used in combination with one or more additional therapies, *e.g*., vaccines, known in the art for the prevention or treatment of an infectious disease, *e.g*., a disease caused by infection with one or more species/strains of bacteria.

As used herein, the terms "manage," "managing," and "management" refer to the beneficial effects that a subject derives from a therapy *(e.g.,* a prophylactic or therapeutic agent), which does not result in a cure of the disease. In certain embodiments, a subject is administered one or more therapies (*e.g*., prophylactic or therapeutic agents, such as a composition of the invention) to "manage" an infectious disease, or a condition or symptom associated therewith, so as to prevent the progression or worsening of disease/disorder.

As used herein, the terms "prevent", "preventing" and "prevention" refer to the prevention of onset of, the recurrence of, or a reduction in one or more symptoms of a disease/disorder (e.g., infection by a bacterium) in a subject as result of the administration of a therapy (*e.g*., a prophylactic or therapeutic composition). As used herein, "prevention" also encompasses prevention of infection by one or more types and/or strains of bacteria associated with the use of the immunoconjugates of the invention as a vaccine.

The term "polysaccharide" and "oligosaccharide" as used herein are used in their broadest sense to refer to saccharides comprising a plurality of repeating units, including, but not limited to saccharides having from 2 to over 2,000 repeating units. Typically, as accepted in the art and as used herein, the term "polysaccharide" refers to a saccharide having from about 50 to about 2,000 or more repeating units. As accepted in the art and as used herein, the term "oligosaccharide" refers to a saccharide having from about 5 to about 40, 45 or 50 repeating units. The repeating unit of a polysaccharide may be a single monosaccharide molecule or may be a disaccharide molecule. In certain embodiments, the repeating unit is 3 or more monosaccharide molecules. In accordance with the methods of the invention, fragments of polysaccharides or oligosaccharides from differing types and/or strains of bacteria may be chemically joined or synthetically synthesized to form a single polysaccharide or oligosaccharide chain comprising multiple epitopes from the multiple types and/or strains of bacteria from which the fragments were originally derived and/or identified; accordingly, the composition of the repeating unit(s) of the polysaccharide or oligosaccharide of the invention need not be constant over the entire saccharide chain. The polysaccharides or oligosaccharides encompassed by the methods of the invention comprise one or more amino sugars. In certain embodiments, said one or more amino sugar is a component of the repeating unit of the polysaccharide or oligosaccharide. In other embodiments, the said one or more amino sugar is not a component of the repeating unit of the polysaccharide or oligosaccharide.

As used herein, the terms "subject" or "patient" are used interchangeably. As used herein, the terms "subject" and "subjects" refers to an animal (*e.g*., mammals). In some embodiments, the subject is a mammal, including non-primates (*e.g*., camels, donkeys, zebras, cows, horses, cats, dogs, rats, and mice) and primates (*e.g*., monkeys, chimpanzees, and humans). In some embodiments, the subject is a non-human mammal. In other embodiments the subject is a human. In certain embodiments, the subject is a human infant, toddler, adolescent, female, or pregnant female.

The term "therapeutic immune response," as used herein, refers to an increase in humoral and/or cellular immunity, as measured by standard techniques, which is directed toward the glycoconjugate. Preferably, but not by way of limitation, the induced level of humoral immunity directed toward glycoconjugate is at least four-fold, eight-fold, or ten-fold, preferably at least 16-fold, greater than the levels of the humoral immunity directed toward the glycoconjugate prior to the administration of the compositions of this invention to the subject. The immune response may also be measured qualitatively, by means of a suitable in vitro or in vivo assay, wherein an arrest in progression or a remission of an infectious disease, or symptoms thereof, in the subject is considered to indicate the induction of a therapeutic immune response.

As used herein, the terms "therapies" and "therapy" can refer to any protocol(s), method(s), and/or agent(s) that can be used in the prevention, treatment, management, or amelioration of a disease/disorder (*e.g*., bacterial infection or a condition or symptom associated therewith). In certain embodiments, the terms "therapies" and "therapy" refer to biological therapy, supportive therapy, and/or other therapies useful in treatment, management, prevention, or amelioration of a disease or condition or symptom(s) associated therewith, an infection or a condition or symptom associated therewith, known to one of skill in the art.

As used herein, the terms "therapeutic agent" and "therapeutic agents" refer to any agent(s) that can be used in the prevention, treatment, management, or amelioration of a disease (*e.g*. bacterial infection or a condition or symptom associated therewith). Preferably, a therapeutic agent is an agent which is known to be useful for, or has been or is currently being used for the prevention, treatment, management, or amelioration of a disease or symptom associated therewith (*e.g*., a bacterial infection or a condition or symptom associated therewith).

As used herein, the terms "treat," "treatment," and "treating" in the context of administration of a therapy to a subject for a disease refers to the eradication, reduction or amelioration of symptoms of said disease/disorder (*e.g*., bacterial disorder).

### 4. BRIEF DESCRIPTION OF THE DRAWINGS:

**FIG. 1** shows a schematic flow diagram of a general method of synthesizing an N-acylated glycoconjugate, where n= number of repeat units, which varies depending on the type of oligo/polysaccharide, and may be 1 to 1,000 or more.
**FIG. 2** shows a schematic flow diagram of a general method of polysaccharide-protein conjugation, for example, preparation of Group A Streptococcus polysaccharide-protein conjugate via N-Pentenoylation. n= number of repeat units, which depends on nature of the polysaccharide or oligosaccharide, and may be 1 to 1,000 or more.
**FIG. 3** depicts the 600 MHz ¹H-NMR spectra of native and modified Group A Streptococcus polysaccharide.
**FIG. 4** shows a schematic flow diagram of a general method of polysaccharide-protein conjugation, for example, preparation of Meningococcal B polysaccharide-protein conjugates via N-pentenoylation, where n= number of repeat units.
**FIG. 5** depicts the 600 MHz ¹H-NMR spectra of modified Meningoccal B polysaccharide.

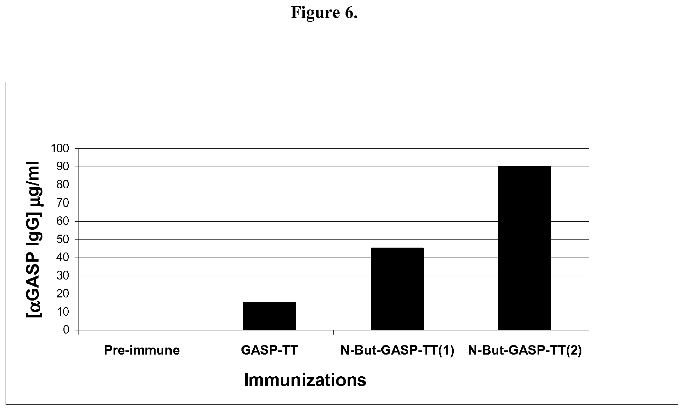

**FIG. 6** shows the immune response against polysaccharides generated by vaccination with group A *Streptococcus* polysaccharide-tetanus toxoid conjugates in BalbC mice. Legends: *Pre-immune:* Serum IgG concentration against group A *Streptococcus* polysaccharide before immunization with conjugates. *GASP-TT:* Serum IgG concentration against group A Streptococcus polysaccharide after immunization with group A *Streptococcus* polysaccharide-protein conjugate, prepared by traditional method (reduction of the native polysaccharide and generation of only one active site per polysaccharide molecule by mild Na-meta-periodate oxidation). *N-But-GASP-TT(1):* Serum IgG concentration against group A *Streptococcus* polysaccharide after immunization with group A *Streptococcus* polysaccharide-protein conjugates, prepared by new method (generation of multiple active site per polysaccharide molecule by N-pentenoylation and oxidation of native polysaccharide). About 5-10% of the GlcNAc residues were pentenoylated. *N-But-GASP-TT(2):* Serum IgG concentration against group A *Streptococcus* polysaccharide after immunization with group A *Streptococcus* polysaccharide-protein conjugates, prepared by new method (generation of multiple active site per polysaccharide molecule by N-pentenoylation and oxidation of native polysaccharide). About 15-20% of the GlcNAc residues were pentenoylated.

### 5. DETAILED DESCRIPTION OF THE INVENTION

This invention generally provides methods of making (i) unsaturated microbial N-acyl derivative oligosaccharides or polysaccharides; (ii) novel immunogenic conjugates derived from the unsaturated N-acyl derivative polysaccharides or oligosaccharides covalently bound to a carrier protein; and (iii) immunogenic glycoconjugate compositions comprising molecules of the invention and a pharmaceutically acceptable carrier. The disclosure further encompasses methods of use of these compositions as vaccines. As disclosed herein, the invention provides methods that can facilitate generation of multiple active sites per oligosaccharide or polysaccharide, in particular for conjugation to one or more proteins, *e.g*., carrier proteins. The invention also provides methods of forming oligosaccharides or polysaccharides with multiple activated sites which, when conjugated with one or more suitable carrier proteins, generate cross-linked glyconjugate vaccines at enhanced efficiency as compared to methods of manufacture previously known in the art. In certain embodiments, the methods of the present invention also generate glycoconjugate vaccines that demonstrate enhanced immunogenicity compared to similar vaccines known in the art. The method presented herein also is applicable to any polysaccharide containing at least one amino sugar, a clear advantage over existing periodate oxidation methods, which require two adjacent free hydroxy groups in the sugar chain.

Oligosaccharides or polysaccharides containing at least one amino sugar having an N-acetyl groups (for example, but not limited to, those comprising one or more of GlcNAc, ManNAc, GalNAc, and/or Sialic acid) are treated to deaceltylate the one or more N-acetyl groups to generate in the oligosaccharide or polysaccharide at least one, preferably multiple, amino-sugar(s) containing a primary amino group. The at least one primary amino group(s) is then substituted with an N-acyl moiety to generate at least one, preferably multiple, active site(s) per polysaccharide or oligosaccharide molecule. To allow subsequent oxidation, the N-acylation is performed using at least a 5-carbon unsaturated aliphatic chain (*e.g*., pentenoylation). The use of unsaturated aliphatic chains greater than 5 carbons in length are also encompassed by the invention (*e.g*., a 6-, 7-, 8-, 9-, 10-, 11-, 12, 13-or 14- or more carbon unsaturated aliphatic chain), provided that the site of unsaturation is not between carbons 1 and 2 or between carbons 2 and 3 of said moiety (See FIG. 1 or, according to Formula I, between the aldehyde group carbon and C₁ of R₁, or between C₁ and C₂ of R₁). Because oxidation can cleave the polysaccharide or oligosaccharide chain, the oxidation methods encompassed by the invention are selected (as described herein and/or as is known in the art) so as to only oxidize the site(s) of unsaturation of the unsaturated aliphatic chain. Such oxidation conditions are normally termed "mild" in the art and may be determined by routine experimentation. Methods of the invention therefore encompass using an oxidizing agent that oxidizes unsaturated groups (*e.g*., the use of O₃ (for Ozonolysis) or H₂O₂, under mild conditions (*e.g*. at a temperature range of about 4 °C to about 27 °C and a pH range of about 4 to about 9.5 in a strongly buffered solution) to generate an active aldehyde (-CHO) group at the site(s) of unsaturation of said alkyl moiety. In certain embodiments, the site of unsaturation of the alkyl moiety is between the terminal two carbons of the alkyl backbone chain. Oxidation of the double bond cleaves the backbone alkyl chain at the site of unsaturation and generates an active aldehyde group at the new alkyl chain terminus. For example, in embodiments wherein the unsaturated aliphatic chain is a C₅ with a single double bond between carbons C₄ and C₅ oxidation will result in the chain of 4 carbons with the aldehyde at C₄. In embodiments wherein the unsaturated aliphatic chain has more than one double bond, *i.e*., sites of unsaturation, the oxidation reaction may affect one or both sites of unsaturation; however, because the reaction cleaves the backbone carbon chain, the aldehyde group will always be at the newly formed aliphatic chain terminus. Accordingly, where the backbone chain comprising the unsaturated group has more than one site of unsaturation, the oxidation reaction may result in unsaturated or saturated N-acyl of differing lengths (dependent on whether one or all of the unsaturated sites were oxidized, respectively, and on the location of the double bonds), each moiety, however, comprising an active aldehyde group at the terminus of the aliphatic backbone chain.

The polysaccharide or oligosaccharide of the invention is conjugated to a carrier protein by any method described herein and/or known in the art (*e.g*., reductive amination) to generate an immunogenic glycoconjugate. Schematic flow diagram of a general method is shown in FIG. 1.

The disclosure relates to microbial unsaturated N-acyl derivative oligosaccharides or polysaccharides of Formula I: 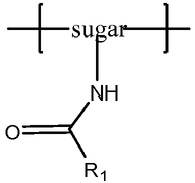 wherein R₁ is an unsaturated C₃ C₄, C₅, C₆, C₇, C₈, C₉, C₁₀ or C₁₁ alkyl moiety comprising at least one unsaturated carbon and the ⁅sugar⁆ represents the said one or more amino sugar of the polysaccharide or oligosaccharide. In certain embodiments, said one or more amino sugar is a component of the repeating unit of the polysaccharide or oligosaccharide. In other embodiments, the said one or more amino sugar is not a component of the repeating unit of the polysaccharide or oligosaccharide. Although the invention encompasses R₁ comprising multiple unsaturated carbons (*i.e*., multiple double bonds), in accordance with the methods provided herein, R₁ needs only to have a single unsaturated carbon (*i.e*., a single double bond; which double bond is not between C₁ and C₂, of R₁). In a certain embodiment of the disclosure R₁ of Formula I is an unsaturated C₃ C₄, C₅, C₆, C₇, C₈, C₉, C₁₀ or C₁₁ alkyl moiety, comprising a single double bond. In a further embodiment of the invention, R₁ of Formula I is an unsaturated C₃ C₄, C₅, C₆, C₇, C₈, C₉, C₁₀, or C₁₁ alkyl moiety, and the position of the double bond is between the terminal two carbons of the aliphatic chain.

In specific embodiments, the unsaturated N-acyl moiety is oxidized to an aldehyde group (at the terminus of the backbone chain of the alkyl moiety) to serve as a linker in conjugating a compound with the carrier protein. The aldehyde group on the N-acyl moiety can then be linked with the carrier protein by any conjugation method known in the art and/or described herein, *e.g*., by reductive amination.

In yet another embodiment, the amino (=NH) group is linked to a sugar residue of the oligosaccharide or polysaccharide at any position, including 1, 2, 3, 4, or 5 of the sugar. The structures of amino sugars are well known in the art, and thus, the position of said linkage may be routinely determined. For example, where the amino sugar is GlcNAc, the N-acetyl group of the sugar is at carbon 2; where the amino sugar is sialic acid, the N-acetyl group of the sugar is at carbon 4.

A non-limiting example of the modified polysaccharides, e.g., N-acyl derivative polysaccharides, of Formula I useful in the present invention is N-pentenoylated derivative polysaccharide, containing at least one N-pentenoyl (CH₂=CH-CH₂-CH₂-CONH-) group as shown in Formula III below: 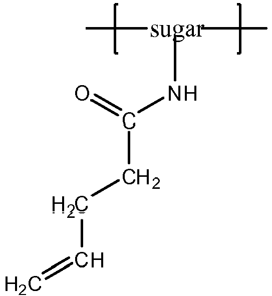 wherein the N-pentenoyl group serves as a linker to conjugate protein.

Any mode of conjugation may be employed to conjugate the modified oligosaccharide or polysaccharide with the carrier protein. One exemplary method, which relies on the presence of terminal vicinal hydroxyl groups to form an active aldehyde group, is described in U.S. Pat. No. 4,356,170 ("the '170 patent). The '170 patent describes the introduction of a terminal aldehyde group into polysaccharide and coupling the aldehyde groups to the protein amino groups by reductive amination. The polysaccharide and the protein are thereby linked through the group as shown below (boxed) in Formula II: 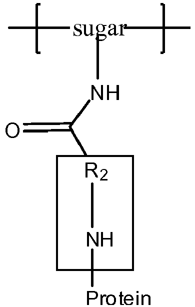 wherein R₂ is a saturated C₂, C₃, C₄, C₅, C₆, C₇, C₈, C₉, or C₁₀ alkyl moiety, and wherein the unboxed NH (boxed) belongs to the one of the primary NH₂ groups of the protein (*e.g*., of lysinyl or arginyl residues). The ⁅sugar⁆ of Formula II represents the said one or more amino sugar of the polysaccharide or oligosaccharide. In certain embodiments, said one or more amino sugar is a component of the repeating unit of the polysaccharide or oligosaccharide. In other embodiments, the said one or more amino sugar is not a component of the repeating unit of the polysaccharide or oligosaccharide.

The resulting N-acylated-polysaccharide-protein conjugates of the invention have been tested in *in vivo* in mice, and have generally been shown to possess improved immunogenic properties as compared with N-propionylated-polysaccharide known in the prior art (*e.g*., those described in the U. S. Patent No. 5,902,586). Accordingly, the vaccines of the invention are expected to be useful against meningitis caused by group B *N. meningitidis* or by *E. coli* K1 organisms. Of particular interest are vaccines for protecting subjects most at risk for bacterial infections (e.g., bacterial meningitis), for example, immunocompromised individuals and infants.

In specific non-limiting embodiments of the invention, it may be desirable to include more than one species of oligosaccharide or polysaccharide, and/or more than one carrier protein, in order to optimize the immune response. Such an approach may be particularly advantageous in the prevention or treatment of infections characterized by the rapid development of mutations that result in evasion of the immune response, *e.g*., protozoal infections. Moreover, an immunogenic glycoconjugate of the invention may include more than one immunogenic/antigenic domain and/or more than one epitope. For example, the invention encompasses multivalent conjugates where at least two differing polysaccharides or oligosaccharides that are specific for differing antigens are conjugated to a single carrier molecule and/or where two differing polysaccharides or oligosaccharides that are specific for differing antigens are combined into a single polysaccharide or oligosaccharide molecule conjugated to a carrier protein. The invention further encompasses conjugates comprising a plurality of polysaccharides or oligosaccharides and or a plurality of carrier proteins. Because the methods of the invention generate at least one, and preferably multiple, active sites per polysaccharide or oligosaccharide molecule, the polysaccharide or oligosaccharide may be covalently bound to the carrier protein at one or more sites; further, the polysaccharide or oligosaccharide may be bound to one or more carrier proteins. Accordingly, in certain embodiments, the conjugate is a lattice of polysaccharide molecules and carrier proteins.

The polysaccharide or oligosaccharide for use in the glycoconjugate compositions of the invention may vary in size. As defined herein, an oligosaccharide for use in the present invention comprises at least 3, at least 4, at least 5, at least 6, at least 7, at least 8, at least 9 or at least 10 repeat units (*e.g*., sugar residues) and preferably from 10 to about 50 repeat units. A polysaccharide, as defined herein, is greater than 50 repeat units and may be as large as about 600 to about 2,000 repeat units or greater. In some cases, large constructs are desirable for enhancement of immunogenicity. The methods of this invention provide for the use of very large polysaccharides because many reactive sites can be introduced into a single polysaccharide.

### 5.1 Polysaccharides and Isolation thereof

Suitable polysaccharides for use in the preferred embodiments include polysaccharides and oligosaccharides from encapsulated bacteria. The polysaccharides and oligosaccharides can be from any source, for example, they can be derived from naturally-occurring bacteria, genetically engineered bacteria, or can be produced synthetically. The polysaccharides and oligosaccharides can be subjected to one or more processing steps prior to activation, for example, purification, functionalization, depolymerization using mild oxidative conditions, deacetylation, and the like. Post processing steps can also be employed, if desired. Any suitable method known in the art for synthesizing, preparing, and/or purifying suitable polysaccharides and oligosaccharides can be employed.

Polysaccharides and oligosaccharides for use in accordance with the methods of the invention include, but are not limited to, pneumococcal polysaccharides of, for example, Serotypes 1, 2, 3, 4, 5, 6B, 7F, 8, 9N, 9V, 10A, 11A, 12F, 14, 15B, 17F, 18C, 19A, 19F, 20, 22F, 23F and 33F; meningococcal polysaccharides of Serotypes A, B, C, W135, and Y, Haemophilus influenzae Type b, polysaccharide polyribosylribitol phosphate, Group B streptococcal polysaccharides of Serotypes III and V and Salmonella typhi Vi polysaccharide. Other polysaccharides of pneuinococcal and Group B streptococcal serotypes, and meningococcal serogroups are also suitable for use herein, as are other typically T-independent polysaccharide and oligosaccharide antigens, for example, polysaccharides or oligosaccharides derived from Group A streptococcus, Staphylococci, Enterococci, Klebsiella pneumoniae, E. coli, Pseudomonas aeruginosa, and Bacillus anthracis. While bacterial polysaccharides and oligosaccharides are particularly preferred, gram (-) bacterial lipopolysaccharides and lipo-oligosaccharides and their polysaccharide and oligosaccharide derivatives, and viral polysaccharides and oligosaccharides can also be employed.

Polysaccharide is isolated from bacterial capsule by methods which are known in the art. For example, in one such method, bacteria such as group B meningococci (strain 981B) are grown at 37°C in a fermenter using 30 g of dehydrated Todd Hewitt Broth (Difco Laboratories, Detroit, Mich.) per liter of distilled water. Prior to fermenter growth, the lyophilized strain is grown initially in a candle jar at 37°C on 5% (v/v) Sheep Blood Agar (Difco Laboratories, Detroit, Mich.) plates. The bacteria are then transferred to 1.0 liter of Todd Hewitt Broth (as above) in an Erlenmeyer flask which is shaken at 37°C for 7 hours at 190 r.p.m. The inoculum is then transferred to the fermenter. After fermenter growth (16 hours) the bacteria are killed by the addition of formalin to a final concentration of 0.75%. The bacteria are removed by continuous centrifugation and the polysaccharide is isolated from the supernatant and purified essentially as described by Bundle et al, J. Biol. Chem., 249, 4797-4801 (1974) except that the protein is extracted by stirring a solution of the crude polysaccharide with cold (4°C) 90% phenol instead of hot (50-60°C). The latter process ensures that a high molecular weight form of the polysaccharide is produced, *e.g*., a high molecular weight form of group B meningococcal polysaccharide (GBMP).

In other specific embodiments, polysaccharides or oligosaccharides may be isolated from bacteria, *e.g., E. coli* (018:K1:H7) (NRCC 4283), by culturing at 37°C in a fermenter containing Brain Heart Infusion (BHI) (Difco Laboratories, Detroit, Mich.) at a concentration of 37 g/liter in distilled water. Working cultures may be started from lyophilized stocks by reconstituting stocks and initial culture in 50 ml of BHI solution (*supra*) in an Erlenmeyer flask which is shaken at 37°C for 7 hours at 200 r.p.m. The culture is then transferred to 1.5 liters of BHI (as above) and grown under the same conditions as described above for 7 hours. The inoculum is then transferred to the fermenter. The isolation and purification of the capsular polysaccharide of bacteria cultured under these conditions, e.g., *E. coli* K1, may be by any method known in the art and/or described herein.

It will be appreciated that the isolation and purification procedures described above are not the only ones which may be utilized, and that other published procedures are available, for example those described by Watson et al, J. Immunol., 81, 331 (1958) and in the above-mentioned U.S. Pat. No. 4,727,136.

### 5.2 Substitution of N-acetyl Groups in Oligosaccharides or Polysaccharides:

N-acetyl groups in native oligosaccharides or polysaccharides can be substituted to provide a reactive amine group in the sialic acid residue parts of the molecule. The substitution can be carried out by any known method, for example, by an alkali, *e.g*., in a basic aqueous medium at elevated temperatures, for example, about 90°C to 110°C, and at a pH of about 13 to 14. In certain embodiments, substitution encompasses deacetylation of the N-acetyl group to form a primary amino group. The basic aqueous medium may comprise an aqueous alkali metal hydroxide solution, e.g., sodium hydroxide of about 2M concentration. Alternatively, hydrazine in aqueous solution can be used. Non-limiting examples of bases which may be used according to this invention are NaOH, KOH, LiOH, NaHCO₃, Na₂CO₃, K₂CO₃, KCN, Et₃N, NH₃, H₂N₂H₂, NaH, NaOMe, NaOEt or KOtBu. Bases such as NaOH, KOH, LiOH, NaH, NaOMe or KOtBu are most effectively used in a range of 0.5 N-5.0 N. Bases such as NaHCO₃, Na₂CO₃, K₂CO₃ and KCN can be used in concentrations as high as their solubilities permit. Bases such as NH₃ or H₂N₂H₂ can be used at nearly any concentration including 100%. Solvents such as water, alcohols (preferably C₁-C₄), dimethylsulfoxide, dimethylformamide or mixtures of these and other organic solvents can be used. Base solutions comprising water are most preferred.

In specific embodiments, the preferred pH range for substitution of the N-acetyl groups of the polysaccharide or oligosaccharide is from about 9 to about 14 with the optimal pH being around 12. The N-substituted polysaccharide thereafter is purified from residual reagents by ultrapurification using membranes or dialysis by standard methods known in the art. The degree of N-acetyl group substitution can vary from a substitution of at least one N-acetyl group up to and including a substitution of 100% of the N-acetyl groups of the oligosaccharide or polysaccharide. In certain embodiments, the degree of N-acetyl group substitution is about 2%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100%. Preferably, 90 to 99% of the native N-acetyl groups are substituted. The substituted oligosaccharides or polysaccharides are recovered, for example, by cooling, neutralizing, purification, and lyophilization. The analysis of extent of N-acetyl substitution and purification of substituted product can be performed by any method known in the art and/or described herein. For example, oligosaccharides and/or polysaccharides can be dialyzed against d.i. water with a Spectra/Por® Membrane MWCO:3,500 for purification/recovery. The extent of N-deacetylation can be analyzed by H¹-NMR at 500 MHz by methods well known in the art.

Prior to the N-acetyl group substitution procedure, native oligosaccharides or polysaccharides have a wide range of average molecular weights, e.g., in the region of about 1,000 to about 1,000,000 Daltons, which average molecular weight is substantially reduced by the N-acetyl group substitution reaction. For example, group B meningococcal polysaccharide (GBMP) has an average molecular weight of about 500,000 to about 800,000 Daltons; after N-acetyl group substitution according to the methods of the invention, fragments of the N-acetyl-substituted polysaccharide are usually produced having an average molecular weight ranging from about 3,000 to about 50,000 Daltons. Full length or fragments of polysaccharides are of use according to the methods of the invention. For example full length polysaccharides or oligosaccharides may be fragmented to produce sizes more suitable for use in conjugate vaccines. For example, N-acylated material of average molecular weight of 10,000 to 40,000 Daltons, preferably, about 10,000 to about 15,000 Daltons, are employed. Fragments of desired size can be obtained by any method known in the art including, *e.g*., centrifugation or filtration methods. In certain embodiments, sized fractions or separation of a desired molecular weight range can be obtained through the use of a fractionating column, *e.g*., by collecting fractions of the eluate of a sizing column (*e.g*., a molecular sieve) to which the starting material has been introduced, *e.g*., N-acylated GBMP material. In certain embodiments, N-acylated material of higher average molecular weight, for example in the region of 30,000 to 40,000 Daltons, is collected for use in the methods of the invention.

The N-acetyl group substituted polysaccharide fragments or full length polysaccharides are then N-acylated to produce the corresponding N-acylated product. The N-acylation can be performed by dissolving the N-acetyl group substituted polysaccharides in an aqueous buffered medium under mildly basic conditions. Preferably, such mild buffered aqueous solutions have a pH of about 7.5 to 9.0. The acyl reagent is then added to the saccharide containing solution and cooled to below 10°C until the reaction is complete. The acyl reagent is selected based on the desired alkyl group at completion of the conjugation, *e.g.,* the desired R₂ in Formula II. The site of unsaturation of the acyl reagent will be oxidized to an active aldehyde and will thus determine the length of R₂. For example, the acyl reagent may be an unsaturated acyl anhydride (for example, acetyl anhydride or propionyl anhydride) or an unsaturated acyl halide (for example, pentenoyl chloride). The reaction is optionally mixed with an alcohol to increase solubility. If desired, the reaction medium can be purified by any method known in the art. A non-limiting example of a purification method that can be utilized is dialysis followed by recovery of the N-acylated product by lyophilization. The reaction is substantially complete within about 10 to 20 hours. The degree of N-acylation of N-acetyl groups is then determined by analytical methods known in the art, *e.g.* ¹H NMR at high resolution, *e.g*., 500 MHz, and is preferably at least 90%, and more preferably about 100%. The N-acylation reaction does not result in any significant molecular weight reduction of the fragments.

### 5.3 Carrier Proteins

The protein(s) to which the polysaccharide is conjugated is chosen so as to be suitable converting a T cell independent immune response to the saccharide component of the vaccine to one that is T cell dependent. In certain embodiments of the invention, the carrier protein can be native toxin or a detoxified toxin (*i.e.* toxoid). Also, non-toxic mutational forms of protein toxins also can be used. Preferably, such mutations retain epitopes of the native toxin. Such mutated toxins have been termed "cross reacting materials", or CRMs. CRM₁₉₇ has a single amino acid change from the active diphtheria toxin and is immunologically indistinguishable from the active toxin. CRM₁₉₇ has been widely used in infants as a component of a *Haemophilus influenzae* conjugate vaccine.

The activated polysaccharide or oligosaccharide is coupled to a protein to yield a conjugate vaccine. Suitable proteins include bacterial toxins that are immunologically effective carriers that have been rendered safe by chemical or genetic means for administration to a subject. Examples include inactivated bacterial toxins such as diphtheria toxoid, CRM.sub.197, tetanus toxoid, pertussis toxoid, E. coli LT, E. coli ST, and exotoxin A from Pseudomonas aeruginosa. Bacterial outer membrane proteins such as, outer membrane complex c (OMPC), porins, transferrin binding proteins, pneumolysis, pneumococcal surface protein A (PspA), pneumococcal adhesin protein (PsaA), or pneumococcal surface proteins BVH-3 and BVH-11 can also be used. Other proteins, such as protective antigen (PA) of Bacillus anthracis, ovalbumin, keyhole limpet hemocyanin (KLH), human serum albumin, bovine serum albumin (BSA) and purified protein derivative of tuberculin (PPD) can also be used. The proteins are preferably proteins that are non-toxic and non-reactogenic and obtainable in sufficient amount and purity that are amenable to the conjugation methods of preferred embodiments. For example, diphtheria toxin can be purified from cultures of Corynebacteria diphtheriae and chemically detoxified using formaldehyde to yield a suitable protein.

Non-limiting examples of carrier proteins include tetanus toxin/toxoid, CRM₁₉₇, Cα, Cβ protein (e.g., from group B Streptococcus, including non-IgA binding C-β protein), diphtheria toxoid, alpha hemolysin, or Panton-Valentine leukocidin (PVL), outer membrane proteins from gram negative bacteria, for example, *Neisseria meningitidis* outer membrane proteins, high molecular weight proteins, P6 and P4 from nontypeable *Haemophilus influenzae,* CD and USPA from *Moraxella catarrhalis*, diphtheria toxin/toxoid, detoxified *Pseudomonas aeruginosa* toxin/toxoid A, cholera toxin/toxoid, pertussis toxin/toxoid, *Clostridium perfringens* exotoxins/toxoid, hepatitis B surface antigen, hepatitis B core antigen, rotavirus VP7 protein, respiratory syncytial virus F, G protein, cholera toxin subunit B, pneumolysoid, pertussis toxoid, synthetic protein containing lysine or cysteine residues, and the like. The carrier protein may be a native protein, a chemically modified protein, a detoxified protein or a recombinant protein. With respect to the protein component, conjugate molecules prepared according to this invention, may be monomers, dimers, trimers and more highly cross-linked molecules.

This invention provides the ability to produce conjugate molecules wherein a carrier protein is linked to an N-acetyl containing polysaccharide or oligosaccharide. The size of the polysaccharide or oligosaccharide may vary greatly. One or a multiplicity of polysaccharides or oligosaccharides may cross-link with one or a multiplicity of proteins. The conjugates of the present invention are preferably lattice structures.

### 5.4 The Conjugate

Many methods are known in the art for conjugating an activated polysaccharide, *i.e*., comprising at least one moiety must be rendered capable of covalently bonding to a protein, to a protein, and are suitable for use herein. For example, U.S. Pat. No. 4,356,170, issued to Jennings, describes conjugation of a polysaccharide comprising an active aldehyde group to a carrier protein by reductive amination using cyanoborohydride.

The methods of the invention allow the generation of at least one, and preferably multiple, active sites (*i.e*., active aldehyde groups) per polysaccharide or oligosaccharide molecule. An activated polysaccharide molecule can react with and form more than one linkage to one or more carrier proteins. Therefore, in certain embodiments, the conjugate product may be a mixture of various crosslinked matrix-type or lattice structures.

After conjugation, the conjugate can be purified by any suitable method. Purification is employed to remove unreacted polysaccharide, protein, or small molecule reaction byproducts. Purification methods include ultrafiltration, size exclusion chromatography, density gradient centrifugation, hydrophobic interaction chromatography, ammonium sulfate fractionation, and the like, as are known in the art. In certain embodiments, no purification may be necessary, or only a minor degree of purification may be desirable. The conjugate can be concentrated or diluted, or processed into any suitable form for use in pharmaceutical compositions, as desired.

### 5.5 PHARMACEUTICAL COMPOSITIONS

Preferably, a composition (e.g., pharmaceutical composition) includes, in admixture, a pharmaceutically acceptable excipient, carrier, or diluent, and one or more of a bioactive agent (e.g., glycoconjugate, oligosaccharide, polysaccharide, polypeptide, or peptide), as described herein, as an active ingredient. The preparation of pharmaceutical compositions that contain bioactive agents as active ingredients is well understood in the art. Typically, such compositions are prepared as liquid solutions or suspensions, however, solid forms suitable for solution in, or suspension in, liquid prior to administration can also be prepared. The preparation can also be emulsified. The active therapeutic ingredient is often mixed with excipients that are pharmaceutically acceptable and compatible with the active ingredient, e.g., a permeation enhancer. Suitable excipients are, for example, water, saline, dextrose, glycerol, ethanol, or the like and combinations thereof. Preferred carriers, excipients, and diluents of the invention comprise physiological saline (i.e., 0.9% NaCl). In addition, if desired, the composition can contain minor amounts of auxiliary substances such as wetting or emulsifying agents, pH-buffering agents, which enhance the effectiveness of the active ingredient.

The compositions of the invention include bulk drug compositions useful in the manufacture of pharmaceutical compositions (e.g., impure or non-sterile compositions) and pharmaceutical compositions (i.e., compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. In certain embodiments, the compositions of the invention comprise an immunogenic amount of at least one immunogenic glycoconjugate and, optionally, a pharmaceutically acceptable carrier. In other embodiments, the compositions of the invention comprise a prophylactically or therapeutically effective amount of at least one immunogenic glycoconjugate and, optionally, a pharmaceutically acceptable carrier.

In a specific embodiment, the term "pharmaceutically acceptable" means physiologically compatible. Preferably, pharmaceutically acceptable means approved by a regulatory agency of the Federal or a state government or listed in the U.S. Pharmacopeia or other generally recognized pharmacopeia for use in animals, and more particularly in humans. The term "carrier" refers to a diluent, excipient, permeation enhancer (in the art as described above), or vehicle with which the therapeutic is administered. Such pharmaceutical carriers include, but are not limited to, sterile liquids, such as water and oils, including those of petroleum, animal, vegetable or synthetic origin, such as peanut oil, soybean oil, mineral oil, sesame oil and the like. Common suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The pharmaceutical compositions of the invention comprising immunogenic glycoconjugates as set forth above are referred to herein as "vaccines." The term vaccine is used to indicate that the compositions of the invention may be used to induce a prophylactic or therapeutic immune response. A vaccine of the invention may comprise a glycoconjugate with a single antigenic domain or epitope, or a glycoconjugate with a plurality of antigenic domains or epitopes. Further, a vaccine may comprise an admixture of glycoconjugates with single or pluralities of antigenic domains or epitopes, or any combination of the foregoing. Pharmaceutical compositions comprising conjugate vaccines of the invention can offer various advantages over conventional vaccines, including enhanced immunogenicity of weakly immunogenic antigens (*e.g*., bacterial polysaccharides or oligosaccharides), potential reduction in the amount of antigen used, less frequent booster immunizations, improved efficacy, preferential stimulation of immunity, or potential targeting of immune responses.

A vaccine composition comprising one or more immunogenic glycoconjugates in accordance with the invention may be administered cutaneously, subcutaneously, intradermally, intravenously, intramuscularly, parenterally, intrapulmonarily, intravaginally, intrarectally, nasally, orally or topically. The vaccine composition may be delivered by injection, particle bombardment, orally or by aerosol.

Vaccine compositions in accordance with the invention may further include various additional materials, such as a pharmaceutically acceptable carrier. Suitable carriers include any of the standard pharmaceutically accepted carriers, such as phosphate buffered saline solution, water, emulsions such as an oil/water emulsion or a triglyceride emulsion, various types of wetting agents, tablets, coated tablets and capsules. An example of an acceptable triglyceride emulsion useful in intravenous and intraperitoneal administration of the compounds is the triglyceride emulsion commercially known as Intralipid.RTM.. Typically such carriers contain excipients such as starch, milk, sugar, certain types of clay, gelatin, stearic acid, talc, vegetable fats or oils, gums, glycols, or other known excipients. Such carriers may also include flavor and color additives or other ingredients.

The vaccine composition of the invention may also include suitable diluents, preservatives, solubilizers, emulsifiers, adjuvants (*e.g*., aluminum phosphate, hydroxide, or sulphate) and/or carriers. Such compositions may be in the form of liquid or lyophilized or otherwise dried formulations and may include diluents of various buffer content (e.g., Tris-HCl, acetate, phosphate), pH and ionic strength, additives such as albumin or gelatin to prevent absorption to surfaces, detergents (e.g., Tween 20, Tween 80, Pluronic F68, bile acid salts), solubilizing agents (e.g. glycerol, polyethylene glycerol), anti-oxidants (e.g., ascorbic acid, sodium metabisulfite), preservatives (e.g., Thimerosal, benzyl alcohol, parabens), bulking substances or tonicity modifiers (e.g., lactose, mannitol, sorbitol), covalent attachment of polymers such as polyethylene glycol to the protein, complexing with metal ions, or incorporation of the material into or onto particulate preparations of polymeric compounds such as polylactic acid, polyglycolic acid, hydrogels, etc. or onto liposomes, microemulsions, micelles, unilamellar or multilamellar vesicles, erythrocyte ghosts, or spheroplasts. Such compositions will influence the physical state, solubility, stability, rate of in vivo release, and rate of in vivo clearance. The choice of compositions will depend on the physical and chemical properties of the vaccine. For example, a product derived from a membrane-bound form of a polysaccharide and/or carrier protein may require a formulation containing detergent. Controlled or sustained release compositions include formulation in lipophilic depots (e.g. fatty acids, waxes, oils). Other embodiments of the compositions of the invention incorporate particulate forms protective coatings, protease inhibitors or permeation enhancers for various routes of administration, including intramuscular, parenteral, pulmonary, nasal and oral.

The compositions of the invention can be formulated as neutral or salt forms. Pharmaceutically acceptable salts include, but are not limited to those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The compositions of the invention, e.g., vaccines, may further comprise one or more adjuvants to enhance immunogenic effectiveness of the composition. The adjuvant used can be any adjuvant known in the art to be suitable for use with polysaccharide-based vaccines (see, *e.g*., U.S. Patent No.:5,773,007). Suitable adjuvants include, but are not limited to oil-in-water emulsion formulations (with or without other specific immunostimulating agents such as muramyl peptides or bacterial cell wall components), such as for example (a) MF59.TM. (WO 90/14837; Chapter 10 in Vaccine design: the subunit and adjuvant approach, eds. Powell & Newman, Plenum Press 1995), containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) RIBI.TM. adjuvant system (RAS), (Ribi Immunochem, Hamilton, Mont.) containing 2% Squalene, 0.2% Tween 80, and one or more bacterial cell wall components such as monophosphorylipid A (MPL), trehalose dimycolate (TDM), and cell wall skeleton (CWS), preferably MPL+CWS (Detox.TM.). Other adjuvants include saponin adjuvants (such as QS21 or Stimulon.TM. (Cambridge Bioscience, Worcester, Mass.) or particles generated therefrom such as ISCOMs (immunostimulating complexes), which ISCOMS may be devoid of additional detergent e.g WO 00/07621); Complete Freund's Adjuvant (CFA) and Incomplete Freund's Adjuvant (IFA); cytokines (such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.); monophosphoryl lipid A (MPL) or 3-O-deacylated MPL (3dMPL) (e.g. GB-2220221, EP-A-0689454, optionally in the substantial absence of alum when used with pneumococcal saccharides e.g. WO 00/56358); combinations of 3dMPL with, *e.g*., QS21 and/or oil-in-water emulsions (e.g. EP-A-0835318, EP-A-0735898, EP-A-0761231); oligonucleotides comprising CpG motifs (Krieg Vaccine 2000, 19, 618-622; Krieg Curr opin Mol Ther2001 3:15-24; Roman et al., Nat. Med, 1997, 3, 849-854; Weiner et al., PNAS USA, 1997, 94, 10833-10837; Davis et al, J. Immunol, 1998, 160, 810-876; Chu et al., J. Exp. Med, 1997, 186, 1623-1631; Lipford et al, Ear. J. Immunol., 1997, 27, 2340-2344; Moldoveami e/ al., Vaccine, 1988, 16, 1216-1224, Krieg et al., Nature, 1995, 374, 546-549; Klinman et al., PNAS USA, 1996, 93, 2879-2883; Ballas et al, J. Immunol, 1996, 157, 1840-1845; Cowdery et al, J. Immunol, 1996, 156, 4570-4575; Halpern et al, Cell Immunol, 1996, 167, 72-78; Yamamoto et al, Jpn. J. Cancer Res., 1988, 79, 866-873; Stacey et al, J. Immunol., 1996, 157,2116-2122; Messina et al, J. Immunol, 1991, 147, 1759-1764; Yi et al, J. Immunol, 1996, 157,4918-4925; Yi et al, J. Immunol, 1996, 157, 5394-5402; Yi et al, J. Immunol, 1998, 160, 4755-4761; and Yi et al, J. Immunol, 1998, 160, 5898-5906; International patent applications WO 96/02555, WO 98/16247, WO 98/18810, WO 98/40100, WO 98/55495, WO 98/37919 and WO 98/52581] i.e. containing at least one CG dinucleotide, where the cytosine is unmethylated); a polyoxyethylene ether or a polyoxyethylene ester (*e.g.* WO 99/52549); a polyoxyethylene sorbitan ester surfactant in combination with an octoxynol (WO 01/21207) or a polyoxyethylene alkyl ether or ester surfactant in combination with at least one additional non-ionic surfactant such as an octoxynol (WO 01/21152); a saponin and an immunostimulatory oligonucleotide (e.g. a CpG oligonucleotide) (WO 00/62800); an immunostimulant and a particle of metal salt (*e.g.* WO 00/23105); a saponin and an oil-in-water emulsion e.g. WO 99/11241; a saponin (e.g QS21)+3dMPL+IM2 (optionally+a sterol) e.g WO 98/57659; and/or other substances that act as immunostimulating agents to enhance the efficacy of the composition.

The pharmaceutical compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of antigen in these formulations can vary widely (*e.g*., from less than about 0.1%, usually at or at least about 2% to as much as 20% to 50% or more by weight), and will be selected primarily based on fluid volumes, viscosities, body weight and the like in accordance with the particular mode of administration selected and the patient's needs. The resulting compositions may be in the form of a solution, suspension, tablet, pill, capsule, powder, gel, cream, lotion, ointment, or aerosol.

Conjugates prepared according to the preferred embodiment are administered to a subject in an immunologically effective dose in a suitable form to treat and/or prevent infectious diseases. The term "subject" as used herein, refers to animals, such as mammals. For example, mammals contemplated include humans, primates, dogs, cats, sheep, cattle, goats, pigs, horses, mice, rats, rabbits, guinea pigs, and the like. The terms "subject", "patient", and "host" are used interchangeably. As used herein, an "immunologically effective" dose of the conjugate vaccine is a dose which is suitable to elicit an immune response. The particular dosage depends upon the age, weight and medical condition of the subject to be treated, as well as on the method of administration. Suitable doses can be readily determined by those of skill in the art.

In practicing immunization protocols for treatment and/or prevention of specified diseases, a therapeutically effective amount of conjugate is administered to a subject. As used herein, the term "effective amount" means the total amount of therapeutic agent (e.g., conjugate) or other active component that is sufficient to show a meaningful benefit to the subject, such as, enhanced immune response, treatment, healing, prevention or amelioration of the relevant medical condition (disease, infection, or the like), or an increase in rate of treatment, healing, prevention or amelioration of such conditions. When "effective amount" is applied to an individual therapeutic agent administered alone, the term refers to that therapeutic agent alone. When applied to a combination, the term refers to combined amounts of the ingredients that result in the therapeutic effect, whether administered in combination, serially or simultaneously. As used herein, the phrase "administering an effective amount" of a therapeutic agent means that the subject is treated with said therapeutic agent(s) in an amount and for a time sufficient to induce an improvement, and preferably a sustained improvement, in at least one indicator that reflects the severity of the disease, infection, or disorder.

The conjugate vaccines of the invention can be administered as a single dose or in a series including one or more boosters. For example, an infant or child can receive a single dose early in life, then be administered a booster dose up to 1, 2, 3, 4, 5, 6, 7, 8, 9, 10 or more years later. The booster dose generates antibodies from primed B-cells, i.e., an anamnestic response. The conjugate vaccine elicits a high primary functional antibody response in infants or children, and is capable of eliciting an anamnestic response following a booster administration, demonstrating that the protective immune response elicited by the conjugate vaccine is long-lived.

Vaccines of the invention can be formulated into liquid preparations for, e.g., oral, nasal, anal, rectal, buccal, vaginal, peroral, intragastric, mucosal, perlinqual, alveolar, gingival, olfactory, or respiratory mucosa administration. Suitable forms for such administration include suspensions, syrups, and elixirs. The conjugate vaccines can also be formulated for parenteral, subcutaneous, intradermal, intramuscular, intraperitoneal or intravenous administration, injectable administration, sustained release from implants, or administration by eye drops. Suitable forms for such administration include sterile suspensions and emulsions. Such conjugate vaccines can be in admixture with a suitable carrier, diluent, or excipient such as sterile water, physiological saline, glucose, and the like. The conjugate vaccines can also be lyophilized. The conjugate vaccines can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "Remington: The Science and Practice of Pharmacy", Lippincott Williams & Wilkins; 20th edition (Jun. 1, 2003) and "Remington's Pharmaceutical Sciences", Mack Pub. Co.; 18.sup.th and 19.sup.th editions (December 1985, and June 1990, respectively), can be consulted to prepare suitable preparations, without undue experimentation. Such preparations can include complexing agents, metal ions, polymeric compounds such as polyacetic acid, polyglycolic acid, hydrogels, dextran, and the like, liposomes, microemulsions, micelles, unilamellar or multilamellar vesicles, erythrocyte ghosts or spheroblasts. Suitable lipids for liposomal formulation include, without limitation, monoglycerides, diglycerides, sulfatides, lysolecithin, phospholipids, saponin, bile acids, and the like. The presence of such additional components can influence the physical state, solubility, stability, rate of in vivo release, and rate of in vivo clearance, and are thus chosen according to the intended application, such that the characteristics of the carrier are tailored to the selected route of administration.

The pharmaceutical compositions of the invention are preferably isotonic with the blood or other body fluid of the recipient. The isotonicity of the compositions can be attained using sodium tartrate, propylene glycol or other inorganic or organic solutes. Sodium chloride is particularly preferred. Buffering agents can be employed, such as acetic acid and salts, citric acid and salts, boric acid and salts, and phosphoric acid and salts. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like.

The pharmaceutical compositions and/or vaccines of the invention can be administered to subject that is at risk for acquiring a disease or disorder (*e.g*., bacterial infection) to prevent or at least partially arrest the development of disease an/or a symptom or complication associated therewith. Amounts effective for therapeutic use will depend on, *e.g*., the antigen composition, the manner of administration, the weight and general state of health of the patient, and the judgment of the prescribing physician. Single or multiple doses of the antigen compositions may be administered depending on the dosage and frequency required and tolerated by the patient, and route of administration.

### 5.5.1 Immunization Regimen

The pharmaceutical compositions and/or vaccines of the invention are administered to a host in a manner that provides for production of selective anti-polysaccharide or anti-oligosaccharide antibodies, preferably, with little or no detectable host autoantibody production.

In particular embodiments, the vaccine compositions described herein are administered serially. First, an immunogenically effective dose of a vaccine of the invention is administered to a subject. The first dose is generally administered in an amount effective to elicit an immune response (e.g., activation T cells). Amounts for the initial immunization generally range from about 0.001 mg to about 1.0 mg per 70 kilogram patient, more commonly from about 0.001 mg to about 0.2 mg per 70 kilogram patient, usually about 0.005 mg to about 0.015 mg per 70 kilogram patient. Dosages from 0.001 up to about 10 mg per patient per day may be used, particularly when the antigen is not administered into the blood stream, such as into a body cavity or into a lumen of an organ. Substantially higher dosages (e.g. 10 to 100 mg or more) are possible in oral, nasal, or topical administration.

After administration of the first vaccine dosage, a therapeutically effective second dose of the vaccine of the invention is administered to the subject after the subject has been immunologically primed by exposure to the first dose. The booster may be administered days, weeks or months after the initial immunization, depending upon the patient's response and condition.

The existence of an immune response to the first vaccine administration may be determined by known methods (e.g. by obtaining serum from the individual before and after the initial immunization, and demonstrating a change in the individual's immune status, for example an immunoprecipitation assay, or an ELISA, or a bactericidal assay, or a Western blot, or flow cytometric assay, or the like) and/or demonstrating that the magnitude of the immune response to the second injection is higher than that of control animals immunized for the first time with the composition of matter used for the second injection (e.g. immunological priming). Immunologic priming and/or the existence of an immune response to the first vaccine administration may also be assumed by waiting for a period of time after the first immunization that, based on previous experience, is a sufficient time for an immune response and/or priming to have taken place--e.g. 2, 4, 6, 10 or 14 weeks. Boosting dosages of the second immunization are typically from about 0.001 mg to about 1.0 mg of antigen, depending on the nature of the immunogen and route of immunization.

In certain embodiments, a therapeutically effective dose of third vaccine composition is administered to the subject after the individual has been primed and/or mounted an immune response to the second vaccine composition. The third booster may be administered days, weeks or months after the second immunization, depending upon the subject's response and condition.

The present invention further contemplates the use of a fourth, fifth, sixth or greater booster immunization, using either the same or differing vaccine formulations.

In certain embodiments, the antigen compositions are administered to a mammalian subject (e.g., human) that is immunologically naive with respect to bacterial source of the polysaccharides or oligosacchraides of the immunoconjugate. In a particular embodiment, the mammal is a human child about five years or younger, and preferably about two years old or younger, and the antigen compositions are administered at any one or more of the following times: two weeks, one month, 2, 3, 4, 5, 6, 7, 8, 9, 10, or 11 months, or one year or 15, 18, or 21 months after birth, or at 2, 3, 4, or 5 years of age.

In preferred embodiments, administration to any mammal is initiated prior to the first sign of disease symptoms, or at the first sign of possible or actual exposure to infection or disease (e.g., due to exposure or infection by Neisseria or E. coli K1).

Pharmaceutical or vaccine compositions can be administered in a manner compatible with the dosage formulation, and in a therapeutically effective amount. The quantity to be administered depends on the subject to be treated, capacity of the subject's immune system to utilize the active ingredient, and degree of modulation required. Precise amounts of active ingredient required to be administered depend on the judgment of the practitioner and are specific for each individual. However, for human infants, a therapeutically, effective dose of the immunogenic glycoconjugate within the pharmaceutical compositions of the present invention comprises about 5 to about 7.5 µg, about 5 to about 10 µg, about 5 to about 12.5 µg, about 5 to about 15 µg, about 5 to about 17.5 µg, about 5 to about 20 µg, about 5 to about 25 µg, about 5 to about 30 µg, about 5 to about 35 µg, about 5 to about 40 µg, about 5 to about 45 µg, or about 5 to about 50 µg; and/or in the range of about 1 to about 1.5 µg, about 1 to about 2 µg, about 1 to about 2.5 µg, about 1 to about 3 µg, about 1 to about 3.5 µg, about 1 to about 4 µg, about 1 to about 5 µg, about 1 to about 6 µg, about 1 to about 7 µg, about 1 to about 8 µg, about 1 to about 9 µg, or about 1 to about 10 µg per kg of body weight.

The pharmaceutical or vaccine compositions of the invention can be administered in combination with various vaccines either currently being used or in development, whether intended for human or non-human subjects. Examples of vaccines for human subjects and directed to infectious diseases include the combined diphtheria and tetanus toxoids vaccine; pertussis whole cell vaccine; the inactivated influenza vaccine; the 23-valent pneumococcal vaccine; the live measles vaccine; the live mumps vaccine; live rubella vaccine; Bacille Calmette-Guerin (BCG) tuberculosis vaccine; hepatitis A vaccine; hepatitis B vaccine; hepatitis C vaccine; rabies vaccine (e.g., human diploid cell vaccine); inactivated polio vaccine; meningococcal polysaccharide vaccine; quadrivalent meningococcal vaccine; yellow fever live virus vaccine; typhoid killed whole cell vaccine; cholera vaccine; Japanese B encephalitis killed virus vaccine; adenovirus vaccine; cytomegalovirus vaccine; rotavirus vaccine; varicella vaccine; anthrax vaccine; small pox vaccine; and other commercially available and experimental vaccines.

### 5.6 Characterization and Demonstration of Therapeutic Utility

Several aspects of the pharmaceutical compositions of the invention are preferably tested *in vitro*, e.g., in a cell culture system, and then *in vivo*, e.g., in an animal model organism, such as a rodent animal model system, for the desired therapeutic activity prior to use in humans. Assays which can be used to assess the likelihood of generating a therapeutic immune response to a particular vaccine composition are well known in the art.

Combinations of prophylactic and/or therapeutic agents can be tested in suitable animal model systems prior to use in humans. Such animal model systems include, but are not limited to, rats, mice, chicken, cows, monkeys, pigs, dogs, rabbits, etc. Any animal system well-known in the art may be used. In a specific embodiment of the invention, combinations of prophylactic and/or therapeutic agents are tested in a mouse model system. Prophylactic and/or therapeutic agents can be administered repeatedly. Several aspects of the procedure may vary such as the temporal regime of administering the prophylactic and/or therapeutic agents, and whether such agents are administered separately or as an admixture.

### 5.7 Toxicity Studies

The toxicity and/or efficacy of the compositions of the present invention can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapies that exhibit large therapeutic indices are preferred. While therapies that exhibit toxic side effects may be used, care should be taken to design a delivery system that targets such agents to the site of affected tissue in order to minimize potential damage to uninfected cells and, thereby, reduce side effects.

The data obtained from animal studies can be used in formulating a range of dosage of the therapies for use in subjects. The dosage of such agents lies preferably within a range of concentrations that include the ED50 with little or no toxicity. The dosage may vary within this range depending upon the dosage form employed and the route of administration utilized. For any therapy used in the method of the invention, the therapeutically effective dose can be estimated initially from animal assays. A dose may be formulated in animal models to achieve an administered concentration range that includes the IC50 (*i.e*., the concentration of the test compound that achieves a half-maximal inhibition of symptoms) as determined in animal models. Such information can be used to more accurately determine useful doses in subjects (e.g., humans).

### 5.8 Kits

The invention also encompasses kits, having a unit dose of the composition present in a storage-stable form, dissolvable or dilutable to the desired dosage together with appropriate packaging and handling devices for convenience of mixing and to maintain sterility prior to instillation. Such a kit can include, for example, a first container containing active ingredient in a stable storage form, either as a unit dose in a stock solution or a unit dose as lyophilized powder; and a second container containing diluent, or solvent and diluent, either separate or combined, the volume of which will provide a unit dose of therapeutic compound in a volume appropriate for administration; means for combining diluent with the stock solution or lyophilized powder; and optionally, means for administering the dose to the patient. Means for transferring diluent to the stock solution or lyophilized powder can include, but are not limited to, syringes or multi-chambered containers having a breachable internal seal separating active ingredient from diluent.

The invention provides a pharmaceutical pack or kit comprising one or more containers filled with the pharmaceutical composition of the invention or a portion thereof. Additionally, one or more other prophylactic or therapeutic agents useful for the treatment of a disease or disorder can also be included in the pharmaceutical pack or kit. The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. In certain embodiments, the compositions of the invention further comprise bulking agents such as sodium chloride, mannitol, polyvinylpyrrolidone and the like, to provide sufficient matter for ease of handling after lyophilization

The present invention provides kits that can be used in the above methods. In one embodiment, a kit comprises one or more pharmaceutical compositions of the invention. In another embodiment, a kit further comprises one or more other prophylactic or therapeutic agents useful for the treatment of an infectious disease or a symptom associated therewith, in one or more containers.

Although the invention has been disclosed by examples of specific embodiments, other embodiments, methods, compositions, active ingredients, indications, compositions and kits will be apparent to those skilled in the art. All such alterations and extensions are included with the invention as disclosed and claimed herein.

### 6. EXAMPLES

### 6.1 EXAMPLE I: Conjugation:

Figure 1 presents a schematic flow diagram of the generalized method of the invention. At least one of the N-acetyl groups (for example, from GlcNAc, ManNAc, GalNAc, and Sailic acid) in a polysaccharide is substituted with an N-acyl moiety to form the compound of Formula I using an alkali, followed by N-pentenoylation (using a 5-carbon unsaturated aliphatic chain). The resulting acylated compound is then oxidized, to generate active aldehyde groups at the unsaturated site of the pentenoyl groups. The oxidized compound is then conjugated with a carrier protein by reductive amination of the activated polysaccharide, which generates the immunogenic glycoconjugate. Immunogenicity of the product may be demonstrated using an animal model (*infra*).

For all experiments, the progress of the conjugation was analyzed with a Biologic system (Bio-Rad) equipped with a superose 12 column. Conjugation of polysaccharide to the antigenic protein was indicated by the progressive increase in a peak, monitored by measurement of UV absorbance at 280 nm, eluting in the void volume of the column. After conjugation was complete, solutions were neutralized to pH 7 with 0.1N HCl and then dialyzed against PBS. The conjugate was purified by passage over a 1.6x60 cm column of Superdex 200 PG (Pharmacia) and eluted with PBS containing 0.01% thimerosal. Fractions corresponding to the void-volume peak were pooled. Carbohydrate and protein content in the conjugate were estimated by the phenol-sulfuric assay of Dubois et al. (51) and the Coomassie assay of Bradford (9).

### 6.2 EXAMPLE II. GROUP A STREPTOCOCCUS (GAS) POLYSACCHARIDE-PROTEIN CONJUGATES:

### Substitution of a portion (35-40%) of the N-Acetyl Groups of GAS polysaccharide:

GAS polysaccharide (30mg/ml) in 0.012N NaOH was treated with NaBH₄ (8 mg/ml) for 75 min with stirring at room temperature ((RT) about 20-25°C). 3N NaOH (1/2 of the volume of 0.012 N NaOH) was added into the reaction mixture with stirring to achieve a final concentration of GAS polysaccharide of 20 mg/ml. The reaction mixture was then maintained at 80°C for 1h. It was then cooled to RT and diluted with water to a final GAS polysaccharide concentration of 4 mg/ml. It was diafiltered against water using 3K regenerated cellulose membrane in Stir-cell. A 15x volume of water was used for diafiltration to achieve a concentration of GAS of 24 mg/ml. The degree of substitution of N-Acetyl Groups with primary amino groups was monitored by ¹H-NMR spectroscopy.

### N-acylation (for example, N-pentenoylation) (15-25%) of N-Acetyl Group-substituted-GAS polysaccharide:

4-pentenoyl chloride (1ml/100mg of polysaccharide) in 1,4-dioxan (1ml/ml of 4-pentenoyl chloride) was added drop wise to a solution of N-Acetyl Group-substituted GAS polysaccharide (24mg/ml) over a period of 75 min with stirring at RT. The pH of the solution was maintained between 6.8 and 9.5 by drop wise addition of 3N NaOH. The pH of the reaction mixture was raised to 12.7 by drop wise addition of 3N NaOH and allowed to stir at RT for 45 min. The pH of the reaction mixture was then decreased to 7.7 by drop wise addition of 1N HCl at RT. The reaction mixture was diluted with water to a final concentration of 4mg/ml GAS polysaccharide and diafiltered using 3K membranes in stircell using water. A 10x volume of water was collected as permeate. Finally, the retentate was concentrated to a polysaccharide concentration of 24 mg/ml and stored at -20°C.

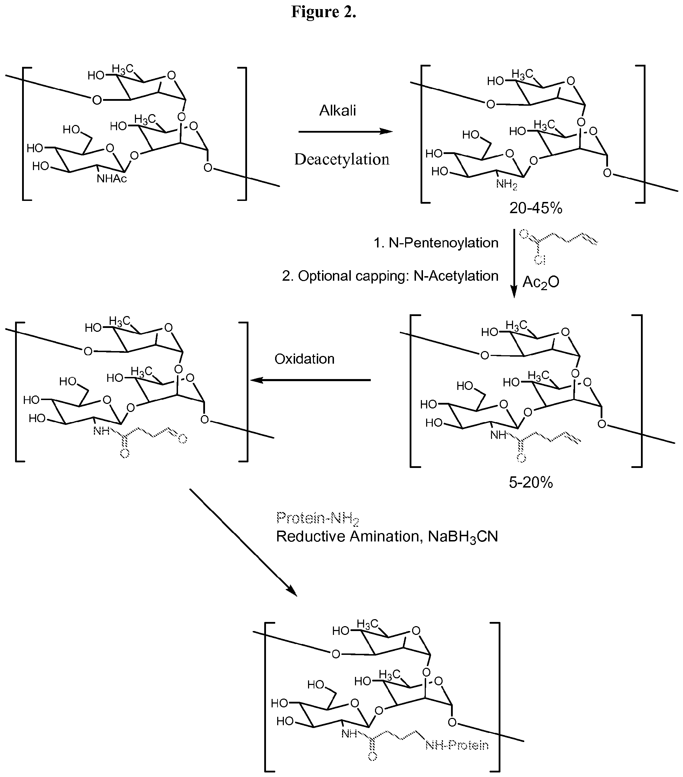

### Optional capping (for example, N-Acetyaltion) of the N-Acetyl Group-substituted (for example, N-pentenoylted)-GAS polysaccharide (See Figure 2):

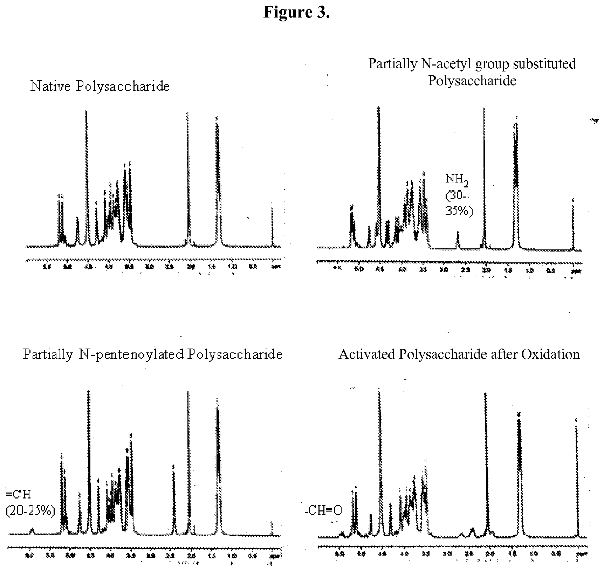

Acetic anhydride (0.6ml/100mg of polysaccharide) was added drop wise to a stirred solution of N-pentenoylated GAS polysaccharide (24mg/ml) over a period of 75 min at RT. The pH of the solution was maintained between 6.8 and 9.5 by drop wise addition of 3N NaOH. The pH of the reaction mixture was then raised to 12.7 by drop wise addition of 3N NaOH and allowed to stir at RT for 60 min. The pH of the reaction mixture was then decreased to 7.7 by drop wise addition of 1N HCl at RT. The reaction mixture was diluted with water to a final concentration of 4mg/ml GAS polysaccharide and diafiltered using 3K membranes in a stircell using water. A 15x volume of water was collected as permeate. Finally, the retentate was concentrated to a polysaccharide concentration of 24 mg/ml and stored at -20°C. Incorporation of pentenoyl groups in the polysaccharides was estimated by 600 MHz ¹H-NMR analyses (See Figure 3).

### Oxidation of the N-Acyl Group-substituted (for example, N-pentenoylted) GAS polysaccharide (See Figure 2):

Methanol (1/2 the volume of the polysaccharide solution) was added slowly to a stirred solution of N-pentenoyl GAS polysaccharide in water (24mg/ml) at RT. The mixture was cooled to between about -15 and -20°C using a dry ice-ethanol bath. Ozone (generated from air using ozonolyzer Ozomax 1) was bubbled through the slowly stirring reaction mixture for 40 min while maintaining the temperature at -15 to -20°C. Nitrogen was then bubbled through the mixture for 10 min to expel the excess ozone. The reaction was then diluted with water to a final concentration of 5 mg/ml polysaccharide and diafiltered using 3K membranes in a stircell using water. 25 x the volume of water was collected as permeate and the retentate was concentrated to a polysaccharide concentration of 20 mg/ml. It was then lyophilized for use in the next step.

### Conjugation to tetanus toxoid/recombinant tetanus toxoid fragment C:

N-butyloxy (N-BuO) GAS polysaccharide (96mg/ml, 1ml) (resulting form the oxidation of an N-pentenoyl GAS polysaccharide) in 0.2mM phosphate buffer (pH 7.7) was added to a solution of tetanus toxoid (conc. 120mg/ml, 0.25 ml) in phosphate buffer (pH 7.7). Sodium cyanoborohydride (40mg) was added to the solution, and the reaction mixture was stirred to achieve a homogeneous mixture. The reaction mixture was incubated at 37 °C for 24h with gentle shaking. After 24h another 16mg of sodium cyanoborohydride was added and the reaction was allowed to proceed for another 48h. After 48h an additional 4 mg of sodium cyanoborohydride was added, and the reaction left at 37°C with shaking for an additional 24h.

A 5% solution of sodium borohydride in 0.05N NaOH (0.5ml) was then added and stirred gently at RT for 1h. Next, a solution of 1N acetic acid (0.72ml in 3 portion) was added with stirring at RT. The reaction was diluted with PBS (pH 7.4) to 32ml and purified in a Labscale TFF system using a 30K membrane. A 30x volume of permeate was collected. The retentate (30ml) was filtered through a 0.2 micron filter and a 10% solution of thimerosal in PBS (0.3ml) was added to the conjugate solution. The conjugate solution was stored at 2-8°C. Compositions of GAS polysaccharide-protein conjugates are shown in Table 1.

### 6.3 Example III. Meningococcal B Polysaccharide-Protein Conjugates:

### N-acylation (for example, N-pentenoylation) (15-25%) of N-Acetyl Group-substituted-Group-substituted Meningococcal B polysaccharide:

4-pentenoyl chloride (1ml/100mg of polysaccharide) in 1,4-dioxan (1ml/ml of 4-pentenoyl chloride) was added drop wise to a solution of N-Acetyl Group-substituted polysaccharide (24mg/ml) over 75 min while stirring at RT. The pH of the solution was maintained between 6.8 and 9.5 by drop wise addition of 3N NaOH. The pH of the reaction mixture was then raised to 12.7 by drop wise addition of 3N NaOH and allowed to stir at RT for 45 min. The pH of the reaction mixture was then decreased to 7.7 by drop wise addition of 1N HCl at RT. The reaction mixture was then diluted with water to a final concentration 4mg/ml of polysaccharide and diafiltered using 3K membranes in a stircell using water. A 10x volume of water was collected as permeate. Finally, the retentate was concentrated to a polysaccharide concentration of 24 mg/ml and stored at -20°C.

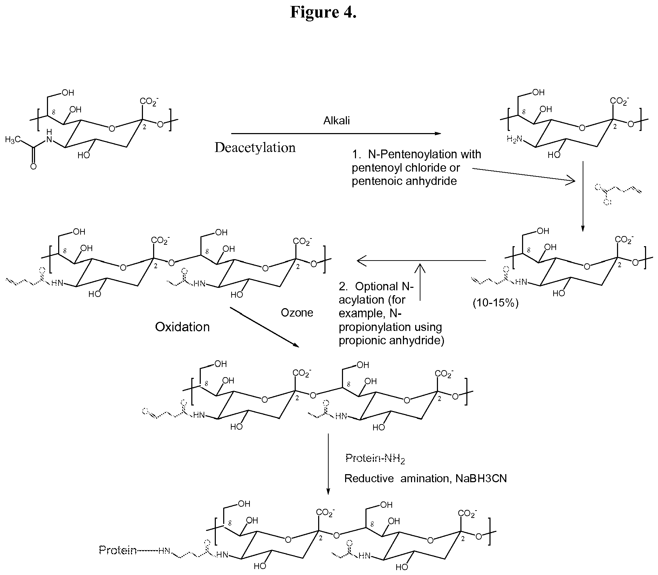

### Optional capping (for example, N-Propionylation) of the N-Acetyl Group-substituted (for example, N-pentenoylted)-Meningococcal B polysaccharide (See Figure 4):

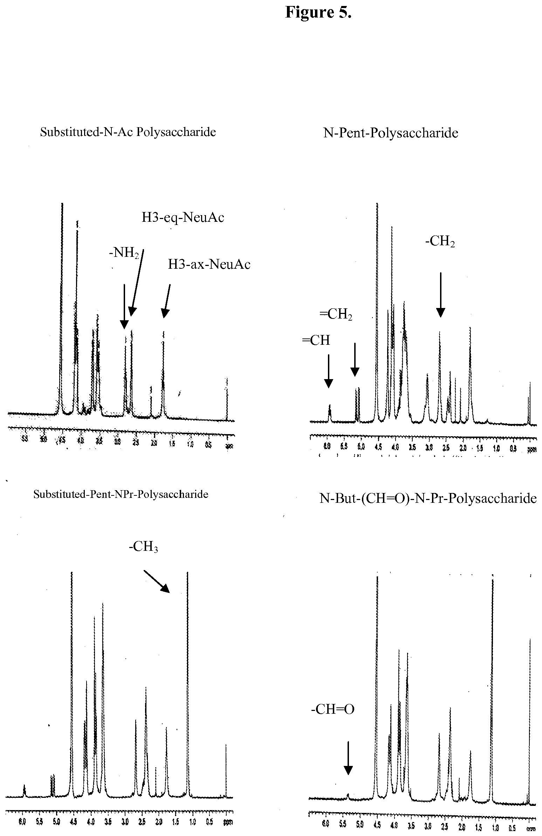

A propionic anhydride-ethanol mixture (2.5:1, 0.84ml/100mg of polysaccharide) was added drop wise to a solution of N-pentenoylated polysaccharide (24mg/ml) over 75 min with stirring at RT. The pH of the solution was maintained between 6.8 and 9.5 by drop wise addition of 3N NaOH. The pH of the reaction mixture was then raised to 12.7 by drop wise addition of 3N NaOH and allowed to stir at RT for 60 min. The pH of the reaction mixture was then decreased to 7.7 by drop wise addition of 1N HCl at RT. The reaction mixture was diluted with water to a final concentration of 4mg/ml polysaccharide and dia-filtered using 3K membrane in a stircell using water. A 15x volume of water was collected as permeate. Finally, the retentate was concentrated to a polysaccharide concentration of 24 mg/ml and stored at -20°C. Incorporation of pentenoyl groups in the polysaccharides was estimated by 600 MHz ¹H-NMR analyses (See Figure 5).

### Oxidation of the N-Acyl Group-substituted (for example, N-pentenoylted) and optionally capped (for example, N-Propionylated)-Group B Meningococcal B polysaccharide (GBMP)

### (See Figure 4):

Methanol (1/2 the volume of the polysaccharide solution) was slowly added to a solution of N-pent-N-Pr GBMP in water (24mg/ml) while stirring at RT. The mixture was then cooled to between about -15 and -20°C using dry ice-ethanol bath. Ozone (generated from air using ozonolyzer Ozomax 1) was bubbled through the gently stirred reaction mixture for 40 min while maintaining the temperature at about -15 to -20°C. Nitrogen was then bubbled through the mixture for 10 min to expel the excess ozone. The reaction was diluted with water to a final concentration of 5 mg/ml polysaccharide and diafiltered using 3K membranes in a stircell with water. 25x volume of water was collected as permeate and the retentate was concentrated to a polysaccharide concentration of 20 mg/ml. It was then lyophilized to use in the next step.

### Conjugation to recombinant Meningococcal Protein (rPorB)/Outer membrane Protein (OMPC) from Meningococcal Bacteria:

Activated N-butanoyloxy-(NbuO)N-Pr GBMP (N-But-[-CH=O]-N-Pr-GBMP, 17mg/ml, 1ml) in HEPES buffer (pH 8.5) was added to solution of rPorB/OMPC (conc. 15mg/ml) in HEPES buffer (pH 8.5). The final concentration of the protein and polysaccharide in the reaction mixture was 1:3. Sodium cyanoborohydride (0.5 times the mass of polysaccharide) was added, and the reaction mixture was stirred to ensure a homogeneous mixture. The reaction mixture was incubated at 37°C for 24h with gentle shaking. After 24h, sodium cyanoborohydride was again added (0.125 times the mass of polysaccharide) and the reaction continued for another 48h. After 48h, a further amount of sodium cyanoborohydride was added (0.032 times the mass of polysaccharide) to the mixture and the reaction maintained at 37°C with shaking for 24h.

A 5% solution of sodium borohydride in 0.05N NaOH (0.2x of the original reaction volume) was then added and stirred gently at RT for 1h. A solution of 1N acetic acid (0.3x of the original reaction volume in 3 portion) was added while stirring at RT. The reaction was diluted with PBS (pH 7.4) to and then purified in a Labscale TFF system using 30K membrane. A 30x volume of permeate was collected. The retentate was filtered through 0.2 micron filter and a 10% solution of thimerosal in PBS (final concentration of thimerosal 0.01 %) was added to the conjugate solution. The conjugate solution was stored at 2-8°C. Compositions of Meningococcal B polysaccharide-protein conjugates are shown in Table 1.

### 6.4 EXAMPLE IV. MENINGOCOCCAL C POLYSACCHARIDE-PROTEIN CONJUGATE:

Substitution of portion of the N-Acetyl groups, acylation, oxidation, and conjugation with protein were done following procedures as described above. Compositions of Meningococcal C polysaccharide-protein conjugates are shown in Table 1.

### 6.5 EXAMPLE V. GROUP B STREPTOCOCCUS TYPE III POLYSACCHARIDE-PROTEIN CONJUGATE:

Following similar experimental procedures as described above, activation and conjugation of Group B Streptococcus type III polysaccharide-protein was performed. Compositions of Group B Streptococcus type III polysaccharide-protein conjugates are shown in Table 1.

**Table 1. Composition of Polysaccharide-Protein Conjugate.**

| **Conjugates** | **Polysaccharide** | **Protein** | **Protein (%) in the Conjugate** | **Polysaccharide (%) in the Conjugate** |
|---|---|---|---|---|
| GASP-TT | Partial-N-But-(CH=O)-GASP | Tetanus Toxoid | 57 | 43 |
| GASP-rTT(C) | Partial-N-But-(CH=O)-GASP | Recombinant Tetanus Toxoid (fragment C) | 70 | 30 |
| Meningococcal B-rPorB | N-But-(CH=O)-N-Pr-MenB | Recombinant Por B | 50 | 50 |
| Meningococcal C-TT | Partial N-But-(CH=O)-MenC | Tetanus Toxoid | 57 | 43 |
| Group B Streptococcus Type III-TT | Partial N-But-(CH=O)-GBSIII | Tetanus Toxoid | 60 | 40 |

### 6.6 EXAMPLE VI. IMMUNIZATION OF BALB C MICE WITH GASP-TT CONJUGATES:

Conjugates (10µg/ml in PBS buffer) were formulated with anhydrogel (1mg/ml). Suspensions of conjugate solution (200 ml, 2µg equivalent of conjugated polysaccharide) in anhydrogel were injected into BalbC mice at intervals of 2 weeks. After 3 consecutive injections, blood samples were collected at 1 week post the last injection. The serum was separated from the sample, and anti-polysaccharide antibodies in the blood serum were quantitated by ELISA. Results of the immune response induced by group A *Streptococcus* polysaccharide-tetanus toxoid conjugates against the polysaccharides in BalbC mice are shown in Figure 6.

### 6.7 EXAMPLE VII. IMMUNIZATION OF CD1 MICE WITH MENGB-RPORB CONJUGATES:

Conjugates (10µg/ml in PBS buffer) were formulated with anhydrogel (1mg/ml). Suspensions of conjugate solution (200 ml, 2µg equivalent of conjugated polysaccharide) in anhydrogel were injected into CD1 mice at intervals of 2 weeks. After 3 consecutive injections, blood samples were collected at 1 week post the last injection and the serum separated. Polysaccharide specific antibodies in the serum were determined by ELISA. Bactericidal activity of the serum was determined by measuring the inhibition of bacterial growth in chocolate agar coated plate in presence of the serum. Bactericidal activity of serum raised Meningococcal B polysaccharide-rPorB conjugates in CD1 mice is shown in Table 2.

Serum bactericidal activity (SBA) of sera generated by 3 separate lots of NPr-(NbuO)-GBMP-rPorB conjugates, lot 1 to lot 3, prepared according to the invention, were significantly higher post bleed at days 42 and 52 than the SBA of the sera generated from NPr-GBMP-rPorB conjugates that were prepared by conventional reductive amination of the sialic acid chain termini (See Table 2). It is believed that the conjugates prepared according to the invention effect improved immune response due to the use of relatively larger size polysaccharide moieties, which are activated at multiple sites along the polysaccharide chain and improve cross-linking of conjugates.

**Table 2. Bactericidal activity of serum raised Meningococcal B polysaccharide-rPorB conjugates in CD1 mice.**

| **Conjugates** | ***SBA day 42** | ***SBA day 52** |
|---|---|---|
| N-Pr-GBMP-rPorB | 1090 | 1949 |
| N-Pr (NBut)-GBMP-rPorB, lot 1 | 4753 | 8073 |
| N-Pr-(NBut)-GBMP-rPorB, lot 2 | 2080 | 6154 |
| N-Pr-(NBut)-GBMP-rPorB, lot 3 | 3373 | 4100 |

| | | |
|---|---|---|
| *Serum bactericidal activity. | | |

## Claims

1. A method of making an immunogenic glycoconjugate comprising
a) substituting at least one N-acetyl group on an antigenic oligosaccharide or polysaccharide comprising one or more N-acetyl substituted amino-sugars with an N-acyl moiety comprising an unsaturated alkyl moiety of greater than 5 carbons, wherein at least one double bond is located between two carbons other than between carbons 1 and 2 or between carbons 2 and 3 of the unsaturated alkyl moiety;
b) contacting the substituted oligosaccharide or polysaccharide with an oxidizing agent to generate at least one active aldehyde group at a site of unsaturation of said alkyl moiety; and
c) conjugating the oligosaccharide or polysaccharide via the at least one active aldehyde group with a carrier protein,
thereby generating an immunogenic glycoconjugate.

2. The method according to claim 1, wherein said substituting the at least one N-acetyl group comprises first de-N-acetylating the at least one N-acetyl group of said antigenic oligosaccharide or polysaccharide to form an oligosaccharide or polysaccharide having at least one amino-sugar comprising a primary amino group and then attaching said N-acyl moiety at said primary amino group.

3. The method according to claim 1 or 2, wherein the N-acetyl group of the oligosaccharide or polysaccharide is substituted with an unsaturated N-acyl group to form formula I: 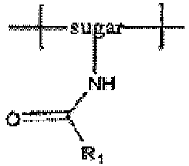 wherein R1 is an unsaturated C₆, C₇, C₈, Cg, C₁₀, or C₁₁ alkyl moiety and {sugar} represents said one or more amino sugars

4. The method according to any one of claims 1-3, wherein the unsaturated alkyl is 6 carbons in length.

5. The method according to any one of claims 1-4, wherein a double bond is located between the terminal two carbons of the unsaturated alkyl moiety.

6. The method according to any one of claims 1-4, wherein said unsaturated alkyl moiety has one double bond, which double bond is located between the terminal two carbons of the alkyl moiety.

7. The method according to claim 1 or 2, wherein said N-acyl moiety is linked to said one or more amino sugars at position 1, 2, 3, 4, or 5 of said one or more amino sugars.

8. The method according claim 1 or 2, wherein the oligosaccharide or polysaccharide is conjugated to the carrier protein via the aldehyde group of the N-acyl moiety by reductive amination.

9. The method according to claim 1 or 2, wherein the carrier protein and the oligosaccharide or polysaccharide of the glycoconjugate are covalently linked through a linkage as follows: 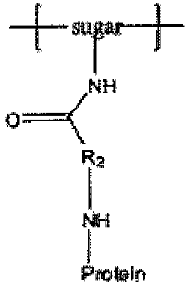 wherein R2 is a saturated C₆, C₇, C₈, C₉, or C₁₀alkyl moiety, wherein the NH of the linkage belongs to a primary NH2 group of the protein, and wherein {sugar} represents said one or more amino sugars.

10. The method according to claim 1, wherein the N-acetyl group is substituted with a N-pentenoyl group, and wherein the N-pentenoyl group serves as a linker in conjugating the compound to the carrier protein.

11. The method according to claim 1 or 2, wherein the N-acelyl group is a moiety of said one or more amino sugars, which one or more amino sugar is one or more of GlcNAc, ManNAc, GalNAc, and Sialic acid.

12. The method according to claim 1, wherein the N-acetyl group is substituted with an N-acyl moiety to form formula I using an alkali.

13. The method according to claim 1 or 2, wherein the carrier protein is tetanus toxin/toxoid, CRM197, outer membrane proteins from gram negative bacteria, P6 and P4 from nontypeable Haemophilus influenzae, CD and USPA from Moraxella catarrhalis, diphtheria toxin/toxoid, detoxified Pseudomonas aeruginosa toxin A, cholera toxin/toxoid, pertussis toxin/toxoid, Clostridium perfringens exotoxins/toxoid, hepatitis B surface antigen, hepatitis B core antigen, rotavirus VP7 protein, or respiratory syncytial virus F and G protein or an active portion thereof

14. The method according to claim 1 or 2, wherein the oligosaccharide or polysaccharide is an oligosaccharide or polysaccharide from a bacterium, wherein the bacterium is a Streptococcus, Staphylococcus, Enterococcus, Bacillus, Corynebacterium, Listeria, Erysipelothrix, Clostridium, Haemophilus, Shigella, Klebsiella, Vibrio cholerae, Neisseria, or Escherichia; or wherein the oligosaccharide or polysaccharide is a capsular polysaccharide derived from Group B Streptococci, Group A Streptococci, Neisseria meningitides, S. pneumoniae, or Escherichia coli; or wherein said capsular polysaccharide is derived from: Group B Streptococci Type Ia, Ib, II, III, V. VI, or VIII, or from Neisseria meningitidis Type B, C, Y, or W135; or from S. pneumoniae Type III, IV, or XIV; or from Escherichia coli KI.

15. The method of claim 1 or 2, wherein the immunogenic glycoconjugate comprises at least two oligosaccharides or polysaccharides conjugated to a single carrier protein and/or at least two carrier proteins conjugated to a single oligosaccharide or polysaccharide.

16. A pharmaceutical composition comprising the immunogenic glycoconjugate according to claim 1, 2 or 15 and a pharmaceutically acceptable carrier; optionally further comprising a plurality of oligosaccharides or polysaccharides; and/or further comprising a plurality of carrier proteins; and/or comprising a plurality of immunogenic glycoconjugates; and/or further comprising one or more adjuvants.

17. The pharmaceutical composition of claim 16 for use in a method of inducing an immune response to one or more types of bacteria, or for use in a method of treating or preventing a bacterial infection in a subject in need thereof.

## Patentansprüche

1. Verfahren zum Herstellen eines immunogenen Glycokonjugats, umfassend:
a) Substituieren von mindestens einer N-Acetylgruppe auf einem antigenen Oligosaccharid oder Polysaccharid, umfassend einen oder mehrere N-Acetyl-substituierte Aminozucker, mit einer N-Acyl-Einheit, umfassend eine ungesättigte Alkyleinheit von mehr als 5 Kohlenstoffatomen, wobei sich mindestens eine Doppelbindung zwischen zwei Kohlenstoffatomen, anders als zwischen den Kohlenstoffatomen 1 und 2 oder zwischen den Kohlenstoffatomen 2 und 3, der ungesättigten Alkyleinheit befindet;
b) Inkontaktbringen des substituierten Oligosaccharids oder Polysaccharids mit einem Oxidationsmittel, um mindestens eine aktive Aldehydgruppe an einer Stelle von Ungesättigtheit der Alkyleinheit zu generieren; und
c) Konjugieren des Oligosaccharids oder Polysaccharids mittels der mindestens einen aktiven Aldehydgruppe mit einem Trägerprotein,
dadurch Generieren eines immunogenen Glycokonjugats.

2. Verfahren gemäß Anspruch 1, wobei Substituieren der mindestens einen N-Acetylgruppe zuerst De-N-acetylieren der mindestens einen N-Acetylgruppe des antigenen Oligosaccharids oder Polysaccharids, um ein Oligosaccharid oder Polysaccharid, aufweisend mindestens einen Aminozucker, umfassend eine primäre Aminogruppe, zu bilden, und dann Anfügen der N-Acyl-Einheit an die primäre Aminogruppe umfasst.

3. Verfahren gemäß Anspruch 1 oder 2, wobei die N-Acetylgruppe des Oligosaccharids oder Polysaccharids mit einer ungesättigten N-Acylgruppe substituiert wird, um Formel I zu bilden: 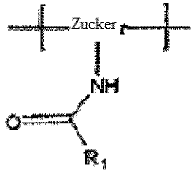 wobei R₁ eine ungesättigte C₆-, C₇-, C₈-, C₉-, C₁₀- oder C₁₁-Alkyleinheit ist und {Zucker} den einen oder die mehreren Aminozucker darstellt.

4. Verfahren gemäß einem der Ansprüche 1-3, wobei das ungesättigte Alkyl eine Länge von 6 Kohlenstoffatomen hat.

5. Verfahren gemäß einem der Ansprüche 1-4, wobei sich eine Doppelbindung zwischen den endständigen zwei Kohlenstoffatomen der ungesättigten Alkyleinheit befindet.

6. Verfahren gemäß einem der Ansprüche 1-4, wobei die ungesättigte Alkyleinheit eine Doppelbindung hat, welche Doppelbindung sich zwischen den endständigen zwei Kohlenstoffatomen der Alkyleinheit befindet.

7. Verfahren gemäß Anspruch 1 oder 2, wobei die N-Acyleinheit mit dem einen oder den mehreren Aminozuckern an Position 1, 2, 3, 4 oder 5 des einen oder der mehreren Aminozucker verknüpft wird.

8. Verfahren gemäß Anspruch 1 oder 2, wobei das Oligosaccharid oder Polysaccharid mit dem Trägerprotein mittels der Aldehydgruppe der N-Acyleinheit durch reduktive Aminierung konjugiert wird.

9. Verfahren gemäß Anspruch 1 oder 2, wobei das Trägerprotein und das Oligosaccharid oder Polysaccharid des Glycokonjugats durch eine Verknüpfung wie folgt kovalent verknüpft werden: 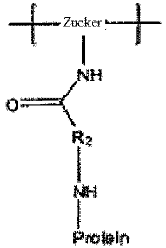 wobei R₂ eine gesättigte C₆-, C₇-, C₈-, C₉- oder C₁₀Alkyleinheit ist, wobei das NH der Verknüpfung zu einer primären NH₂-Gruppe des Proteins gehört und wobei {Zucker} den einen oder die mehreren Aminozucker darstellt.

10. Verfahren gemäß Anspruch 1, wobei die N-Acetylgruppe mit einer N-Pentenoylgruppe substituiert wird und wobei die N-Pentenoylgruppe beim Konjugieren der Verbindung mit dem Trägerprotein als Linker dient.

11. Verfahren gemäß Anspruch 1 oder 2, wobei die N-Acetylgruppe eine Einheit des einen oder der mehreren Aminozucker ist, welcher eine oder welche mehreren Aminozucker eines oder mehrere von GlcNAc, ManNAc, GalNAc und Sialinsäure ist oder sind.

12. Verfahren gemäß Anspruch 1, wobei die N-Acetylgruppe unter Verwendung eines Alkali mit einer N-Acyleinheit substituiert wird, um Formel I zu bilden.

13. Verfahren gemäß Anspruch 1 oder 2, wobei das Trägerprotein Tetanus-Toxin/Toxoid, CRM197, äußere Membranproteine von gramnegativen Bakterien, P6 und P4 von nichttypisierbarem *Haemophilus influenzae,* CD und USPA von *Moraxella catarrhalis*, Diphtherie-Toxin/Toxoid, entgiftetes *Pseudomonas-aeruginosa-Toxin* A, Cholera-Toxin/Toxoid, *Pertussis*-Toxin/Toxoid, *Clostridium-perfringens-*Exotoxine/Toxoid, Hepatitis-B-Oberflächenantigen, Hepatitis-B-Kernantigen, Rotavirus-VP7-Protein oder Respiratory-syncytial-Virus-F- und -G-Protein oder ein aktiver Anteil davon ist.

14. Verfahren gemäß Anspruch 1 oder 2, wobei das Oligosaccharid oder Polysaccharid ein Oligosaccharid oder Polysaccharid von einem Bakterium ist, wobei das Bakterium ein *Streptococcus*, *Staphylococcus*, *Enterococcus*, *Bacillus*, *Corynebacterium*, *Listeria, Erysipelothrix, Clostridium, Haemophilus, Shigella, Klebsiella, Vibrio cholerae, Neisseria* oder *Escherichia* ist; oder wobei das Oligosaccharid oder Polysaccharid ein Kapselpolysaccharid, abgeleitet von Gruppe-B-*Streptococci*, Gruppe-A-*Streptococci*, *Neisseria meningitides*, *S. pneumoniae* oder *Escherichia coli* ist; oder wobei das Kapselpolysaccharid von: Gruppe-B-*Streptococci* Typ Ia, Ib, II, III, V, VI oder VIII, oder von *Neisseria meningitidis* Typ B, C, Y oder W135; oder von *S. pneumoniae* Typ III, IV oder XIV; oder von *Escherichia coli* K1 abgeleitet ist.

15. Verfahren nach Anspruch 1 oder 2, wobei das immunogene Glycokonjugat mindestens zwei Oligosaccharide oder Polysaccharide, konjugiert mit einem einzigen Trägerprotein, und/oder mindestens zwei Trägerproteine, konjugiert mit einem einzigen Oligosaccharid oder Polysaccharid, umfasst.

16. Pharmazeutische Zusammensetzung, umfassend das immunogene Glycokonjugat gemäß Anspruch 1, 2 oder 15 und einen pharmazeutisch verträglichen Träger; wahlweise weiterhin umfassend eine Mehrzahl von Oligosacchariden oder Polysacchariden; und/oder weiterhin umfassend eine Mehrzahl von Trägerproteinen; und/oder umfassend eine Mehrzahl von immunogenen Glycokonjugaten; und/oder weiterhin umfassend einen oder mehrere Adjuvantien.

17. Pharmazeutische Zusammensetzung nach Anspruch 16, zur Verwendung in einem Verfahren zum Induzieren einer Immunantwort auf einen oder mehrere Typen von Bakterien oder zur Verwendung in einem Verfahren zum Behandeln oder Verhindern einer bakteriellen Infektion bei einem Subjekt, das dessen bedarf.

## Revendications

1. Procédé de préparation d'un glycoconjugué immunogène comprenant:
a) substituer au moins un groupe N-acétyle sur un oligosaccharide ou un polysaccharide antigène comprenant un ou plusieurs sucres aminés substitués N-acétyle par une fraction N-acyle comprenant une fraction alkyle insaturée de plus de 5 carbones, où au moins une double liaison est située entre deux carbones autres qu'entre les carbones 1 et 2 ou entre les carbones 2 et 3 de la fraction alkyle insaturée;
b) mettre l'oligosaccharide ou le polysaccharide substitué en contact avec un agent oxydant pour produire au moins un groupe aldéhyde actif à un site d'insaturation de ladite fraction alkyle; et
c) conjuguer l'oligosaccharide ou le polysaccharide par l'intermédiaire du au moins un groupe aldéhyde actif avec une protéine porteuse,
produisant ainsi un glycoconjugué immunogène.

2. Procédé selon la revendication 1, dans lequel ledit fait de substituer le au moins un groupe N-acétyle comprend premièrement dé-N-acétyler le au moins un groupe N-acétyle dudit oligosaccharide ou polysaccharide antigène pour former un oligosaccharide ou un polysaccharide ayant au moins un sucre aminé comprenant un groupe amino primaire et puis attacher ladite fraction N-acyle audit groupe amino primaire.

3. Procédé selon la revendication 1 ou 2, dans lequel le groupe N-acétyle de l'oligosaccharide ou du polysaccharide est substitué par un groupe N-acyle insaturé pour former la formule I: 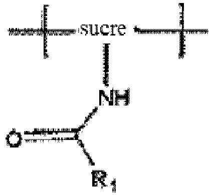 dans laquelle R1 est une fraction alkyle en C₆, C₇, C₈, C₉, C₁₀ ou C₁₁ insaturée et {sucre} représente ledit un ou plusieurs sucres aminés.

4. Procédé selon l'une quelconque des revendications 1-3, dans lequel l'alkyle insaturé fait 6 carbones de longueur.

5. Procédé selon l'une quelconque des revendications 1-4, dans lequel une double liaison est située entre les deux carbones terminaux de la fraction alkyle insaturée.

6. Procédé selon l'une quelconque des revendications 1-4, dans lequel ladite fraction alkyle insaturée a une double liaison, laquelle double liaison est située entre les deux carbones terminaux de la fraction alkyle.

7. Procédé selon la revendication 1 ou 2, dans lequel la fraction N-acyle est liée audit un ou auxdits plusieurs sucres aminés à la position 1, 2, 3, 4 ou 5 dudit un ou desdits plusieurs sucres aminés,

8. Procédé selon la revendication 1 ou 2, dans lequel l'oligosaccharide ou polysaccharide est conjugué à la protéine porteuse par l'intermédiaire du groupe aldéhyde de la fraction N-acyle par amination réductrice.

9. Procédé selon la revendication 1 ou 2, dans lequel la protéine porteuse et l'oligosaccharide ou polysaccharide du glycoconjugué sont liés d'une manière covalente par une liaison comme suit: 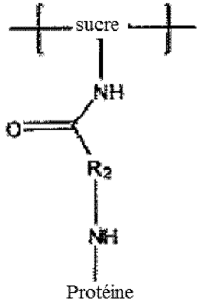 où R2 est une fraction alkyle en C₆, C₇, C₈, C₉ ou C₁₀ saturée, où le NH de la liaison appartient à un groupe NH₂ primaire de la protéine, et où {sucre} représente ledit un ou lesdits plusieurs sucres aminés.

10. Procédé selon la revendication 1, dans lequel le groupe N-acétyle est substitué par un groupe N-penténoyle, et dans lequel le groupe N-penténoyle sert en tant que lieur pour conjuguer le composé à la protéine porteuse.

11. Procédé selon la revendication 1 ou 2, dans lequel le groupe N-acétyle est une fraction dudit un ou desdits plusieurs sucres aminés, lequel un ou lesquels plusieurs des sucres aminés sont un ou plusieurs d'entre GlcNAc, ManNAc, GalNAc et acide sialique.

12. Procédé selon la revendication 1, dans lequel le groupe N-acétyle est substitué par une fraction N-acyle pour former la formule I en utilisant un alcali.

13. Procédé selon la revendication 1 ou 2, dans lequel la protéine porteuse est une toxine/toxoïde de tétanos, CRM 197, protéines de la membrane externe de bactéries à Gram négatif, P6 et P4 d'*Haemophilus influenzae* non typable , CD et USPA de *Moraxella catarrhalis,* toxine/toxoïde de diphtérie, toxine A détoxifiée de *Pseudomonas aeruginosa,* toxine/toxoïde du choléra, toxine/toxoïde de *pertussis,* exotoxines/toxoïde de *Clostridium perfringens,* antigène de surface de l'hépatite B, antigène coeur de l'hépatite B, protéine VP7 de rotavirus, ou bien les protéines F et G du virus respiratoire syncytial ou une partie active de ceux-ci.

14. Procédé selon la revendication 1 ou 2, dans lequel l'oligosaccharide ou polysaccharide est un oligosaccharide ou un polysaccharide d'une bactérie, où la bactérie est *Streptococcus, Staphylococcus, Enterococcus, Bacillus, Corynebacterium, Listeria, Erysipelothrix, Clostridium, Haemophilus, Shigella, Klebsiella, Vibrio cholerae, Neisseria,* ou *Escherichia;* ou bien dans lequel l'oligosaccharide ou le polysaccharide est un polysaccharide capsulaire dérivé de *Streptococci* du Groupe B, *Streptococci* du Groupe A, *Neisseria meningitidis, S. pneumoniae,* ou *Escherichia coli;* ou bien dans lequel ledit polysaccharide capsulaire est dérivé de: *Streptococci* du Groupe B, Ia, Ib, II, III, V, VI, ou VIII, ou bien de *Neisseria meningitidis* Type B, C, Y ou W135; ou bien de *S. pneumoniae* Type III, IV, ou XIV; ou bien *d'Escherichia coli* Kl.

15. Procédé selon la revendication 1 ou 2, dans lequel le glycoconjugué immunogène comprend au moins deux oligosaccharides ou polysaccharides conjugués à une seule protéine porteuse et/ou au moins deux protéines porteuses conjuguées à un seul oligosaccharide ou polysaccharide.

16. Composition pharmaceutique comprenant le glycoconjugué immunogène selon la revendication 1, 2 ou 15 et un véhicule pharmaceutiquement acceptable, comprenant optionnellement en outre une pluralité d'oligosaccharides ou de polysaccharides; et/ou comprenant en outre une pluralité de protéines porteuses; et/ou comprenant une pluralité de glycoconjugués immunogènes; et/ou comprenant en outre un ou plusieurs adjuvants.

17. Composition pharmaceutique selon la revendication 16, à utiliser dans une méthode d'induction d'une réponse immune à un ou plusieurs types de bactéries, ou à utiliser dans une méthode de traitement ou de prévention d'une infection bactérienne chez un sujet en ayant besoin.
